(19) Europäisches Patentamt
European Patent Office
Office européen des brevets

(11) EP 3 508 492 A1

(12) EUROPEAN PATENT APPLICATION

(43) Date of publication:
10.07.2019 Bulletin 2019/28

(51) Int Cl.:
*C07H 17/08* (2006.01) *A61K 31/7048* (2006.01)
*A61P 31/04* (2006.01)

(21) Application number: 18150671.8

(22) Date of filing: 08.01.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD TN

(71) Applicant: Universität Zürich
8006 Zürich (CH)

(72) Inventors:
- GADEMANN, Karl
  8600 Dübendorf (CH)
- BERG, Regina
  68623 Lampertheim (DE)
- MEIER, Andrea
  8610 Uster (CH)

(74) Representative: Schulz Junghans Patentanwälte PartGmbB
Großbeerenstraße 71
10963 Berlin (DE)

# (54) TIACUMICIN DERIVATIVES AND THEIR USE AS ANTIBIOTICS

(57) The invention relates to a compound according to formula (1)

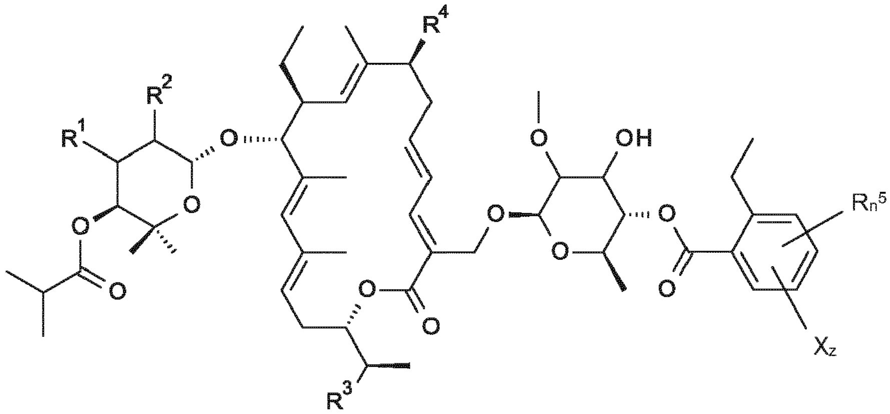

(1),

wherein $R^1$ to $R^4$ and X are independently from each other a small functional group and $R^5$ is -OH, or $-O-L_n-R^a_p-R^b_q-L_t-R^a_r-R^b_s-R^e$, $-O-L_n-R^b_q-R^d-L_t-R^b_s-R^e$ with L being an alkyl linker, $R^a$ being carbonyl, carboxyl or carboxamide, $R^b$ being a cyclic moiety, $R^d$ being a polyether linker and $R^e$ being a small functional end group, fluorescent dye or antibiotic with the proviso that the compound is not fidaxomicin.

Furthermore, the invention relates to the use of the compound in the treatment of disease and the use in treating infections and/or bacterial infections caused by drug resistant bacteria.



Printed by Jouve, 75001 PARIS (FR)

## Description

Field of the invention:

The present invention relates to novel macrolide compounds and their use as antibiotics.

Background of the invention

Members of the tiacumicin family of natural products are macrolide compounds with antibacterial and antifungal activity. Although they have been isolated in 1972, the first commercially available antibiotic from this class, fidaxomicin, has only been approved by the FDA in 2011. Fidaxomicin has been reported to show antibacterial activity against gram-positive bacteria in the micromolar range and has been approved as a drug for the treatment of *Clostridium difficile*-associated diarrhea.

A limitation of the members of the tiacumicin family including fidaxomicin, are their poor pharmacokinetic properties. As fidaxomicin is not systemically absorbed, it can only serve as a topical treatment for *Clostridium difficile* infections in the gut. Thus, the high antibacterial potential of tiacumicin antibiotics against a wide variety of pathogenic bacteria has not been exploited yet. Derivative compounds with an improved pharmacokinetic profile, in particular with an improved solubility and absorbability, would allow for systemic administration of the drug and could be used for a much wider variety of infections. Facing an enormous spread of resistant bacterial strains, a generation of semisynthetically modified tiacumicins could be used as drugs of last resort for treatment of problematic infections such as tuberculosis.

Based on the above mentioned state of the art, the objective of the present invention is to provide derivatives of tiacumicin compounds with an improved pharmacokinetic profile to be used in the treatment of bacterial infections. This objective is attained by the claims of the present specification.

Description of the invention

According to a first aspect of the invention a compound characterized by a general formula (1) is provided.

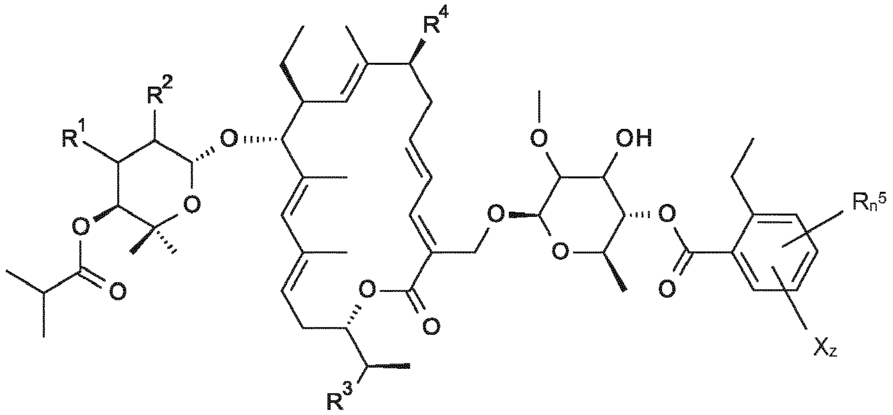

(1),

- wherein

- $R^1$ and $R^2$ are independently from each other $C_{1-4}$-alcohol, -OH, -O-$R^k$ or -O-C(=O)-$R^k$, or $R^1$ and $R^2$ together are -O-C(=O)-O-, with $R^k$ being a $C_{1-4}$-alkyl or $C_{2-4}$-alkenyl,

- $R^3$ and $R^4$ are independently from each other $C_{1-6}$-alcohol, -OH, $C_{1-6}$-alkoxy, silyl ether, in particular tert-butyldimethylsilyl ether (TBS), -$NR^iH$, -$NR^iR^j$, $P_{1-6}$-phosphane, phenol, -O-C(=O)-$R^i$, -OCN, with $R^i$ and $R^j$ being independently from each other a $C_{1-4}$-alkyl or $C_{2-4}$-alkenyl, wherein $R^3$ and $R^4$ are unsubstituted or substituted by $C_{1-4}$-alkyl or a $C_{1-4}$-alkyl substituted with at least one -F, -Cl, -Br or -I,

- n of $R_n^5$ is 1 or 2, and each $R^5$ is independently from each other $R^5$
  -OH, or
  -O-$L_n$-$R^a_p$-$R^b_q$-$L_tR^a_r$-$R^b_s$-$R^e$, in particular -O-$L_n$-$R^a_p$-$R^b_q$-$L_t$-$R^a_r$-$R^e$ or -O-$L_n$-$R^a_p$-$R^b_q$-$L_t$-$R^b_s$-$R^e$, or
  -O-$L_n$-$R^b_q$-$R^d$-$L_tR^b_s$-$R^e$
  with

- n, p, q, r, s and t being independently from each other 0 or 1,
- each L being independently from each other L selected from $C_{1}$-$_{12}$-alkyl,
- $R^a$ being independently from each other $R^a$ -(C=O)-, -(C=O)-NH-, -(C=O)-$NR^e$-, -NH-(C=O)-, -(C=O)-O- or-O-(C=O)-,
- $R^b$ being independently from each other $R^b$ a three to six membered heterocycle or heteroaryl, a $C_{3-6}$- cycloalkyl or $C_{6-14}$-aryl,
- $R^d$ is a polyether linker comprising m elements -$C_u$-alkyl-O-, wherein u is independently selected for each element from an integer between 1 and 4 and m is an integer between 1 and 12, in particular 1 and 8,
- $R^e$ being -H, -$C_{1-4}$-alkyl, -$C_{2-4}$-alkenyl, -$C_{2-4}$-alkynyl, -OH, -COOH, -$NH_2$, -$N_3$, -$SO_2F$, -F, -Cl, -Br, -I, a dye, in particular a fluorescent dye, more particularly fluorescein, or an antibiotic, in particular ciprofloxacin or rifampicin,

- z of $X_z$ is 1 or 2, and each $X_z$ is independently from each other $X_z$ -CN, -$CF_3$, - $CH_2F$, -$CHF_2$, -$CH_2$-$CF_3$, -$CH_2$-$CH_2F$, -$CH_2$-$CHF_2$, -$OR^h$, -$SR^h$, -$SOR^h$, -$SO_2R^h$, $SO_2$-NH-$R^h$, -F, -Cl, -Br or -I, wherein $R^h$ is a $C_{1}$-$_{4}$-alkyl or a $C_{1}$-$_{4}$-alkyl substituted with at least one -F, -Cl, -Br or -I,

with the proviso that the compound is not fidaxomicin.

In certain embodiments, the compound of formula (1) is not a compound with $R^1$ and $R^2$ together being -O-C(=O)-O- and $R^3$, $R^4$ and $R^5$ being -OH, n being 2, X being Cl and z being 2.

The residues $R^1$ and $R^2$ may be together -O-C(=O)-O-. This means that $R^1$ and $R^2$ form a ring structure, wherein the first O atom of the -O-C(=O)-O- moiety binds at the position $R^1$ and the last O atom of the the -O-C(=O)-O- moiety binds at the position $R^2$.

The linker L may be a linear or branched alkyl, in particular a linear alkyl. If n and t are both 1, both linker L may be a linear alkyl, both may be a branched alkyl or one L may be a linear alkyl and the other L may be a branched alkyl.

In certain embodiments, L is selected from $C_{1-12}$alkyl, in particular $C_{1-6}$-alkyl, more particularly $C_{1-3}$-alkyl.

The residue $R^5$ contributes to an improved pharmacokinetic of the compounds according to the invention in comparison to fidaxomicin itself. The compounds according to the invention are effective against bacteria such as Mycobacterium tuberculosis. Furthermore, the compounds according to the invention show an increased solubility compared to fidaxomicin.

The elements that form the polyether linker $R^d$ are independently selected from -alkyl-O-moieties such as -$CH_2$-O- (u=1), -$(CH_2)_2$-O- (u=2), -$(CH_2)_3$-O- (u=3), -$(CH_2)_4$-O- (u=4) or branched alkyl-O- moieties such as -isopropyl-O- (u=3), -*tert*-butyl-O- (u=4), -isobutyl-O-(u=4) or -*sec*-butyl-O- (u=4).

The polylinker $R^d$ may comprise a mix of linear and branched, only linear or only branched - alkyl-O- moieties, in particular only linkear alkyl-O- moieties, wherein u may be the same for all elements or different for all or some elements.

The polylinker $R^d$ may comprise several identical elements, for example five -$(CH_2)_2$-O-moieties. In this case, m equals 5 and u for each element is 2.

The polylinker $R^d$ may comprise several different elements, for example one -$CH_2$-O- moiety and four -$(CH_2)_2$-O-moieties. In this case, m equals 5 and u is 1 for the first element and u is 2 for the second to fifth element.

In certain embodiments, the elements that form the polyether linker $R^d$ are selected from - $CH_2$-O-, -$(CH_2)_2$-O-, -$(CH_2)_3$-O-, -$(CH_2)_4$-O-, -isopropyl-O-, -*tert*-butyl-O-, -isobutyl-O- or -*sec*-butyl-O-.In certain embodiments, the elements that form the polyether linker $R^d$ are selected from $CH_2$-O-, -$(CH_2)_2$-O-, -$(CH_2)_3$-O- or -$(CH_2)_4$-O-, in particular $CH_2$-O-, -$(CH_2)_2$-O- or -$(CH_2)_3$-O-, more particularly $CH_2$-O- or -$(CH_2)_2$-O-.

The compounds according to the present invention may be fidaxomicin derivatives coupled to a dye. Such compounds are for example useful for studying cellular uptake, localization or distribution in a biological assay or model system or in a living organism.

In certain embodiments, $R^e$ is a fluorescent dye, in particular fluorescein.

The compounds according to the present invention may be fidaxomicin derivatives coupled to an antibiotic. Such compounds may be effective against a broader variety of bacteria compared to fidaxomicin or the other antibiotic alone. Moreover, the solubility of such compounds compared to the solubility of fidaxomicin may be enhanced.

In certain embodiments, $R^e$ is an antibiotic, in particular rifampicin or ciprofloxacin.

In certain embodiments, $R^e$ is a fluorescent dye or an antibiotic, in particular flurorescein, rifampicin or ciprofloxacin.

The term "substituted" refers to the addition of a substituent group to a parent compound.

"Substituent groups" can be protected or unprotected and can be added to one available site or to many available sites in a parent compound. Substituent groups may also be further substituted with other substituent groups and may be attached directly or by a linking group such as an alkyl or hydrocarbyl group to a parent compound. "Substituent groups" amenable herein include, without limitation, halogen, oxygen, nitrogen, sulphur, hydroxyl, alkyl, alkenyl, alkynyl, acyl (-C(O)$R^\alpha$), carboxyl (-C(O)O$R^\alpha$), aliphatic groups, alicyclic groups, alkoxy, substituted oxy (-O$R^\alpha$), aryl, aralkyl, heterocyclic radical, heteroaryl, heteroarylalkyl, amino (-N($R^\beta$)($R^\gamma$)), imino (=N$R^\beta$), amido (-C(O)N($R^\beta$)($R^\gamma$) or

$-N(R^{\beta})C(O)R^{\alpha}$), hydrazine derivates ($-C(NH)NR^{\alpha}R^{\beta}$), tetrazole ($CN_4H_2$), azido ($-N_3$), nitro ($-NO_2$), cyano (-CN), isocyano (-NC), cyanato (-OCN), isocyanato (-NCO), thiocyanato (-SCN); isothiocyanato (-NCS); carbamido ($-OC(O)N(R^{\beta})(R^{\gamma})$ or $-N(R^{\beta})C(O)OR^{\alpha}$), thiol ($-SR^{\beta}$), sulfinyl ($-S(O)R^{\beta}$), sulfonyl ($-S(O)_2R^{\beta}$), sulfonamidyl ($-S(O)_2N(R^{\beta})(R^{\gamma})$or $-N(R^{\beta})S(O)_2R^{\beta}$) and fluorinated compounds $-CF_3$, $-OCF_3$, $-SCF_3$, $-SOCF_3$ or $-SO_2CF_3$. Wherein each $R^{\alpha}$, $R^{\beta}$ and $R^{\gamma}$ is, independently, H or a further substituent group with a preferred list including without limitation, H, alkyl, alkenyl, alkynyl, aliphatic, alkoxy, acyl, aryl, heteroaryl, alicyclic, heterocyclic and heteroarylalkyl.

A $C_1$-$C_6$ alkyl in the context of the present invention signifies a saturated linear or branched hydrocarbon having 1, 2, 3, 4, 5 or 6 carbon atoms. Non-limiting examples for a $C_1$-$C_6$ alkyl include methyl, ethyl, propyl, butyl, isopropyl, n-hexyl, 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl, pent-4-inyl, 3-methyl-2-pentyl, and 4-methyl-2-pentyl.

A cyclic alkyl in the context of the present invention signifies a cyclic saturated hydrocarbon such as cyclopropyl, cyclobutyl, cyclopentyl or cyclohexyl.

The term alkenyl in the context of the present invention refers to a straight or branched hydrocarbon chain moiety containing up to 10 carbon atoms and having at least one carbon-carbon double bond. Examples of alkenyl groups include, without limitation, ethenyl, propenyl, butenyl, 1-methyl-2-buten-1-yl, dienes such as 1,3-butadiene and the like. Alkenyl groups typically include from 2 to about 10 carbon atoms, more typically from 2 to about 6 carbon atoms. Alkenyl groups as used herein may optionally include further substituent groups.

The term alkynyl in the context of the present invention refers to a straight or branched hydrocarbon moiety containing up to 10 carbon atoms and having at least one carbon-carbon triple bond. Examples of alkynyl groups include, without limitation, ethynyl, 1-propynyl, 1-butynyl, and the like. Alkynyl groups typically include from 2 to about 10 carbon atoms, more typically from 2 to about 6 carbon atoms. Alkynyl groups as used herein may optionally include further substituent groups.

The term alcohol in the context of the present invention refers to a hydroxyl-substituted alkyl moiety. A $C_6$-alcohol signifies a hydroxyl-substituted $C_6$-alkyl such as $-(CH_2)_6-OH$. Non-limiting examples of hydroxy-substituted alkyl include $-CH_2OH$, $-(CH_2)_2OH$, $-(CH_2)_3OH$, $-CH_2CH(OH)CH_3$, $-(CH_2)_4OH$, $-CH(CH_2OH)CH_2CH_3$, $-CH_2CH(CH_2OH)CH_3$, $-CH(OH)(CH_2)_2OH$, $-CH_2CH(OH)CH_2OH$, $-CH_2CH(OH)(CH_2)_2OH$ and $-CH_2CH(CH_2OH)_2$.

The term silyl ether in the context of the present invention refers to a $-O-Si-R^xR^yR^z$ moiety, wherein $R^x$, $R^y$ and $R^z$ are independently from each other selected from linear or branched $C_{1-6}$-alkyl, $C_{4-8}$-cyclo-alkyl or phenyl, Non limiting examples of silyl ether include trimethylsilyl ether, triethylsilyl ether, *tert*-butyldimethylsilyl ether, *tert*-butyldiphenylsilyl ether and triisopropylsilyl ether.

The term heterocycle in the context of the present invention signifies a cyclic $C_3$ to $C_8$ hydrocarbon moiety comprising one or several nitrogen, oxygen and/or sulphur atoms. Examples for heterocycles includes aziridine, azetidine, piperazine, piperidine, imidazole, triazole, tetrazole and tetrahydrothiophene.

The term ciprofloxacin in the context of the present invention signifies the antibiotic compound 1-cyclopropyl-6-fluoro-4-oxo-7-(piperazin-1-yl)-quinoline-3-carboxylic acid (CAS 85721-33-1).

The term rifampicin in the context of the present invention signifies the antibiotic compound (7S,9E,11S,12R,13S,14R,15R,16R,17S,18S,19E,21Z)-2,15,17,27,29-pentahydroxy-11-methoxy-3,7,12,14,16,18,22-heptamethyl-26-{(E)-[(4-methylpiperazin-1-yl)imino]methyl}-6,23-dioxo-8,30-dioxa-24-azatetracyclo[23.3.1.14,7.05,28]triaconta-1(28),2,4,9,19,21,25(29), 26-octaen-13-yl acetate (CAS 13292-46-1).

The term fluorescein in the context of the present invention signifies the antibiotic compound 3',6'-dihydroxyspiro[isobenzofuran-1(3*H*),9'-[9*H*]xanthen]-3-one (CAS 2321-07-5).

The term fidaxomicin in the context of the present invention signifies the antibiotic compound 3-(((6-Deoxy-4-*O*-(3,5-dichloro-2-ethyl-4,6-dihydroxybenzoyl)-2-*O*-methyl-β-D-mannopyranosyl)oxy)-methyl)-12(*R*)-[(6-deoxy-5-*C*-methyl-4-*O*-(2-methyl-1-oxopropyl)-β-D-lyxo-hexopyranosyl)oxy]-11(*S*)-ethyl-8(*S*)-hydroxy-18(*S*)-(1(R)-hydroxyethyl)-9,13,15-trimethyloxacyclooctadeca-3,5,9,13,15-pentaene-2-one (CAS 873857-62-6).

The term aryl in the context of the present invention signifies a cyclic aromatic $C_5$-$C_{10}$ hydrocarbon. Examples of aryl include, without being restricted to, phenyl, naphthyl and heteroaryl. A heteroaryl in the context of the present invention is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of the invention additionally may be substituted by one or more alkyl groups.

In certain embodiments the compound is characterized by general formula (2),

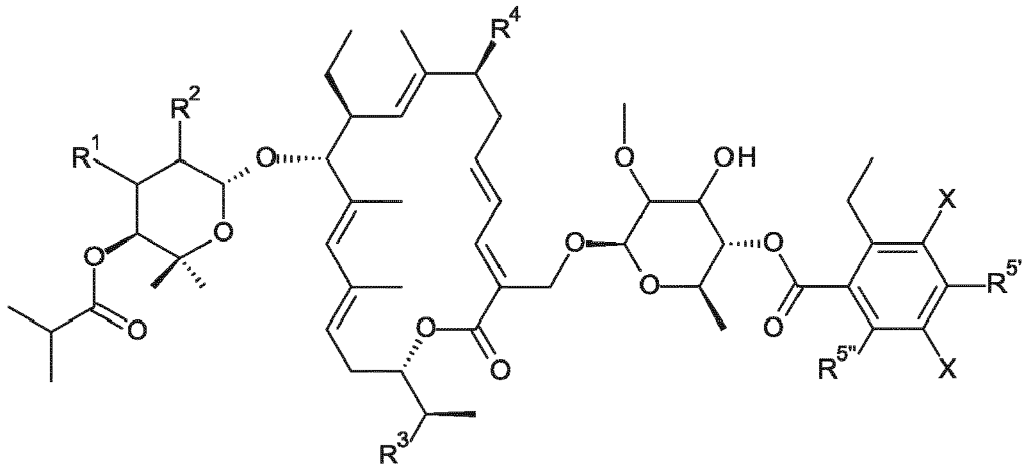

(2),

with $R^{5'}$ and $R^{5''}$ having independently from each other the same meaning as defined for $R^5$ and $R^1$,$R^2$, $R^3$, $R^4$, $R^5$ and X having the same meaning as defined above.

In certain embodiments, $R^5$ and $R^{5''}$ are independently from each other -OH, -O-L-$R^a$-$R^e$, -O$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, - O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$.

In certain embodiments, $R^{5'}$ and $R^{5''}$ are independently from each other -OH, -O-L-$R^a$-$R^e$, - O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$.

In certain embodiments, $R^{5'}$ and $R^{5''}$ are independently from each other -OH, -O-L-$R^a$-$R^e$, -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$.

In certain embodiments, $R^{5'}$ and $R^{5''}$ are independently from each other -OH, -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$L-$R^e$, or -O-$R^d$L-$R^e$.

In certain embodiments, $R^{5'}$ and $R^{5''}$ are independently from each other -OH, -O-L-$R^a$-$R^b$-$R^e$, -O-L-$R^b$-L-$R^b$-$R^e$, -O-$R^d$-$R^e$, -O-L-$R^b$-$R^d$-L-$R^e$-O-L-$R^a$-$R^b$-$R^a$-$R^e$, -O-L-$R^b$-$R^b$-$R^e$, or -O-$R^d$-L-$R^e$.

In certain embodiments, $R^{5'}$ and $R^{5''}$ are independently from each other -OH, -O-L-$R^a$-$R^b$-$R^e$, -O-L-$R^b$-L-$R^b$-$R^e$, -O-$R^d$-$R^e$, or -O-L-$R^b$-$R^d$-L-$R^e$.

In certain embodiments, $R^{5'}$ and $R^{5''}$ are independently from each other -OH, -O-L-$R^a$-$R^e$, -O-L-$R^b$-L-$R^b$-$R^e$, or -O-$R^d$-L-$R^b$-$R^e$.

In certain embodiments, one of $R^{5'}$ and $R^{5''}$, in particular $R^{5'}$, is selected from -O-L-$R^a$-$R^e$, -O$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, - O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^e$, -O-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, - O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O$R^d$-L-$R^b$-$R^e$, in particular

one of $R^{5'}$ and $R^{5''}$ , in particular $R^{5'}$, is selected from -O-L-$R^a$-$R^e$, -O-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^e$, -O-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$.

In certain embodiments, one of $R^{5'}$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$, in particular

one of $R^{5'}$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O$R^d$-$R^e$, -O-L-$R^b$-$R^d$-L-$R^e$, -O-$R^d$-L-$R^e$, or -O-

$R^d$-L-$R^b$-$R^e$.

In certain embodiments, one of $R^{5'}$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$ and the other one of $R^{5'}$ and $R^{5''}$, in particular $R^{5''}$, is -OH or both $R^{5'}$ and $R^{5''}$ are independently from each other selected from -O-L-$R^8$-$R^e$, - O-L-$R^8$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$, in particular

one of $R^{5'}$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$.

In certain embodiments, one of $R^{5'}$ and $R^{5''}$, in particular $R^{5'}$, is selected from -O-L-$R^a$-$R^b$-$R^e$, - O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$, in particular

one of $R^{5'}$ and $R^{5''}$, in particular $R^{5'}$, is selected from -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or - O-$R^d$-L-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$.

In certain embodiments, one of $R^{5'}$ and $R^{5''}$, in particular $R^{5'}$, is selected from -O-L-$R^a$-$R^b$-$R^e$, - O-L-$R^b$-L-$R^b$-$R^e$, -O-$R^d$-$R^e$, -O-L-$R^b$-$R^d$-L-$R^e$, -O-L-$R^a$-$R^b$-$R^a$-$R^e$, -O-L-$R^b$-$R^b$-$R^e$, or -O-$R^d$-L-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^b$-$R^e$, -O-L-$R^b$-L-$R^b$-$R^e$, -O-$R^d$-$R^e$, -O-L-$R^b$-$R^d$-L-$R^e$, -O-L-$R^a$-$R^b$-$R^a$-$R^e$, -O-L-$R^b$-$R^b$-$R^e$, or -O-$R^d$-L-$R^e$, in particular

one of $R^{5'}$ and $R^{5''}$, in particular $R^{5'}$, is selected from -O-L-$R^a$-$R^b$-$R^e$, -O-L-$R^b$-L-$R^b$-$R^e$, -O-$R^d$-$R^e$, -O-L-$R^b$-$R^d$-L-$R^e$, -O-L-$R^a$-$R^b$-$R^a$-$R^e$, -O-L-$R^b$-$R^b$-$R^e$, or -O-$R^d$-L-$R^e$ and the other one of $R^{5'}$ and $R^{5''}$, in particular $R^{5''}$, is -OH, more particularly both $R^{5'}$ and $R^{5''}$ are independently from each other selected from -O-L-$R^a$-$R^b$-$R^e$, -O-L-$R^b$-L-$R^b$-$R^e$, -O-$R^d$-$R^e$, -O-L-$R^b$-$R^d$-L-$R^e$, -O-L-$R^a$-$R^b$-$R^a$-$R^e$, -O-L-$R^b$-$R^b$-$R^e$, or -O-$R^d$-L-$R^e$.

In certain embodiments, one of $R^{5'}$ and $R^{5''}$, in particular $R^{5'}$, is selected from -O-L-$R^a$-$R^b$-$R^e$, - O-L-$R^b$-L-$R^b$-$R^e$, -O-$R^d$-$R^e$, or -O-L-$R^b$-$R^d$-L-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^b$-$R^e$, -O-L-$R^b$-L-$R^b$-$R^e$, -O-$R^d$-$R^e$, or -O-L-$R^b$-$R^d$-L-$R^e$, in particular one of $R^{5'}$ and $R^{5''}$, in particular $R^{5'}$, is selected from -O-L-$R^a$-$R^b$-$R^e$, -O-L-$R^b$-L-$R^b$-$R^e$, -O-$R^d$-$R^e$, or -O-L-$R^b$-$R^d$-L-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^b$-$R^e$, -O-L-$R^b$-L-$R^b$-$R^e$, -O-$R^d$-$R^e$, or -O-L-$R^b$-$R^d$-L-$R^e$.

In certain embodiments, one of $R^{5'}$ and $R^{5''}$ , in particular $R^{5'}$, is selected from -O-L-$R^a$-$R^e$, -O-L-$R^b$-L-$R^b$-$R^e$, or -O-$R^d$-L-$R^b$-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^e$, -O-L-$R^b$-L-$R^b$-$R^e$, or -O-$R^d$-L-$R^b$-$R^e$, in particular

one of $R^{5'}$ and $R^{5''}$, in particular $R^{5'}$, is selected from -O-L-$R^a$-$R^e$, -O-L-$R^b$-L-$R^b$-$R^e$, or -O-$R^d$-L-$R^b$-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^e$, -O-L-$R^b$-L-$R^b$-$R^e$, or -O-$R^d$-L-$R^b$-$R^e$.

In certain embodiments, $R^1$ and $R^2$ are independently from each other -OH, or -O-C(=O)-$R^k$, or $R^1$ and $R^2$ together are -O-C(=O)-O- with $R^k$ being a $C_{1-4}$-alkyl, in particular isopropyl.

In certain embodiments, $R^1$ and $R^2$ are independently from each other -OH or -O-C(=O)-$R^k$, with $R^k$ being a $C_{1-4}$-alkyl, in particular isopropyl.

In certain embodiments, $R^1$ and $R^2$ are -OH.

In certain embodiments, $R^3$ and $R^4$ are independently from each other -OH or $-O-(CH_2)_{1-4}-CH_3$.

In certain embodiments, $R^3$ and $R^4$ are independently from each other -OH, $-O-CH_2-CH_3$ or - $OCH_3$.

In certain embodiments, $R^3$ and $R^4$ are -OH.

In certain embodiments, $R^a$ of $R^5$ is -(C=O)-, -(C=O)-NH-, -NH-(C=O)-, -(C=O)-O-.

In certain embodiments, $R^a$ of $R^5$ is -(C=O)-NH-, -NH-(C=O)-, -(C=O)-O-.

In certain embodiments, $R^a$ of $R^5$ is -(C=O)-NH-, -NH-(C=O)-.

In certain embodiments, $R^a$ of $R^5$ is -(C=O)-, -(C=O)-O-.

In certain embodiments, $R^a$ of $R^5$ -(C=O)-.

In certain embodiments, L is $C_{1-6}$-alkyl.

In certain embodiments, L is $C_{1-3}$-alkyl.

In certain embodiments, $R^b$ of $R^5$ is a five to six membered heterocycle, a $C_{5-6}$-cycloalkyl or $C_6$-aryl.

In certain embodiments, $R^b$ of $R^5$ is a five to six membered heterocycle or $C_6$-aryl.

In certain embodiments, $R^b$ of $R^5$ is independently selected from piperazine, piperidine, tetrazole, triazole, imidiazole or phenyl.

In certain embodiments, $R^b$ of $R^5$ is a five to six membered heterocycle.

In certain embodiments, $R^b$ of $R^5$ is independently selected from piperazine, piperidine, tetrazole, triazole or imidiazole

In certain embodiments, $R^b$ of $R^5$ is independently selected from piperazine, piperidine or triazole.

In certain embodiments, $R^e$ of $R^5$ is -H, -$C_{1-2}$-alkyl, -$C_{2-4}$-alkenyl, allyl, -OH, -COOH, -$NH_2$, -$N_3$, -$SO_2F$, ciprofloxacin, fluorescein or rifampicin.

In certain embodiments, $R^e$ of $R^5$ is -H, $C_{1-2}$-alkyl, -OH, -COOH, -$NH_2$, -$N_3$ or -$SO_2F$.

In certain embodiments, $R^e$ of $R^5$ is -H, $C_{1-2}$-alkyl, -OH, -COOH or -$NH_2$.

In certain embodiments, m is 4 to 8.

In certain embodiments, m is 5 to 7.

In certain embodiments, the elements of $R^d$ are independently selected from -$(CH_2)_{1-4}$-O-.

In certain embodiments, the elements of $R^d$ are independently selected from -$(CH_2)_{1-2}$-O-.

In certain embodiments, each element of $R^d$ -$(CH_2)_2$-O-.

In certain embodiment, the first element of $R^d$ is -$CH_2$-O- and the other elements of $R^d$ are - $(CH_2)_2$-O-.

In certain embodiments, m is 4 to 8 and the elements of $R^d$ are independently selected from - $(CH_2)_{1-4}$-O-.

In certain embodiments, m is 4 to 8 and the elements of $R^d$ are independently selected from - $(CH_2)_{1-2}$-O-.

In certain embodiments, m is 4 to 8 and each element of $R^d$ -$(CH_2)_2$-O-.

In certain embodiments, m is 4 to 8 and the first element of $R^d$ is -$CH_2$-O- and the other elements of $R^d$ are -$(CH_2)_2$-O-.

In certain embodiments, m is 5 to 7 and the elements of $R^d$ are independently selected from - $(CH_2)_{1-4}$-O-.

In certain embodiments, m is 5 to 7 and the elements of $R^d$ are independently selected from - $(CH_2)_{1-2}$-O-.

In certain embodiments, m is 5 to 7 and each element of $R^d$ -$(CH_2)_2$-O-.

In certain embodiments, m is 5 to 7 and the first element of $R^d$ is -$CH_2$-O- and the other elements of $R^d$ are -$(CH_2)_2$-O-.

In certain embodiments, z of $X_z$ is 1 or 2 and each $X_z$ is independently from each other $X_z$ - $CF_3$, -O-$CF_3$, -S-$CF_3$, -SO-$CF_3$, -$SO_2CF_3$, $SO_2$-NH-$CF_3$, -F, -Cl, -Br or -I.

In certain embodiments, z of $X_z$ is 1 or 2 and each $X_z$ is independently from each other $X_z$ -F, -Cl, -Br or -I.

In certain embodiments, z of $X_z$ is 1 or 2 and $X_z$ is -Cl.

In certain embodiments, z of $X_z$ is 2 and each $X_z$ is independently from each other $X_z$ -$CF_3$, - O-$CF_3$, -S-$CF_3$, -SO-$CF_3$, -$SO_2CF_3$, $SO_2$-NH-$CF_3$, -F, -Cl, -Br or -I.

In certain embodiments, z of $X_z$ is 2 and each $X_z$ is independently from each other $X_z$ -F, -Cl, -Br or -I.

In certain embodiments, z of $X_z$ is 2 and $X_z$ is -Cl.

In certain embodiments, the solubility in phosphate-buffered water (pH = 7) is at least 40 $\mu$g/ml.

In certain embodiments, the solubility in phosphate-buffered water (pH = 7) is at least 80 $\mu$g/ml.

In certain embodiments, the solubility in phosphate-buffered water (pH = 7) is >90 $\mu$g/ml.

In certain embodiments, the solubility in phosphate-buffered water (pH = 7) is > 150 $\mu$g/ml.

In certain embodiments, the solubility in phosphate-buffered water (pH = 7) is > 400 $\mu$g/ml.

In certain embodiments, the solubility in phosphate-buffered water (pH = 7) is > 1000 $\mu$g/mL.

In certain embodiments, the solubility in phosphate-buffered water (pH = 7) is > 1500 $\mu$g/mL

In certain embodiments, one of $R^{5'}$ and $R^{5''}$, in particular $R^{5'}$, is selected from -O-L-$R^a$-$R^e$, -O$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, - O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^{5'}$ and $R^{5''}$, in particular $R^{5''}$, is -OH or both $R^{5'}$ and $R^{5''}$ are independently from each other selected from -O-L-$R^b$-$R^e$, -O-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, - O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$, in particular

one of $R^{5'}$ and $R^{5''}$, in particular $R^{5'}$, is selected from -O-L-$R^a$-$R^e$, -O-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^e$, -O-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$, wherein

$R^1$ and $R^2$ are independently from each other -OH or -O-C(=O)-$R^k$, with $R^k$ being a $C_{1-4}$-alkyl, in particular isopropyl,

$R^3$ and $R^4$ are independently from each other -OH or -O-$(CH_2)_{1-4}$-$CH_3$,

$R^a$ of $R^5$ is -(C=O)-, -(C=O)-NH-, -NH-(C=O)-, -(C=O)-O-,

$R^b$ of $R^5$ is a five to six membered heterocycle or $C_6$-aryl,

$R^e$ of $R^5$ is -H, $C_{1-2}$alkyl, -OH, -COOH, $-NH_2$, $-N_3$ or $-SO_2F$,
m is 4 to 8 and the the elements of $R^d$ are independently selected from $-(CH_2)_{1-2}$-O-,
z of $X_z$ is 2 and each $X_z$ is independently from each other $X_z$ -F, -Cl, -Br or -I.

In certain embodiments, one of $R^{5'}$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^{5'}$ and $R^{5''}$ , in particular $R^{5''}$, is -OH or both $R^{5'}$ and $R^{5''}$ are independently from each other selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$L-$R^e$, or -O-$R^d$-L-$R^b$-$R^e$, in particular
one of $R^5$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O-$R^d$-$R^e$, -O-L-$R^b$-$R^d$-L-$R^e$, -O-$R^d$-L-$R^e$, or -O-$R^d$-L-$R^b$-$R^e$ and the other one of $R^5$ and $R^{5''}$, in particular $R^{5''}$, is -OH, more particularly
both $R^{5'}$ and $R^{5''}$ are independently from each other selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^b$-$R^b$-$R^e$, -O-L-$R^b$-$R^e$, -O-L-$R^b$-L-$R^a$-$R^e$, -O-L-$R^b$-L-$R^b$-$R^e$, -O$R^d$-$R^e$, -O-L-$R^b$-$R^d$-L-$R^e$, -O-$R^d$-L-$R^e$, or -O-$R^d$-L-$R^b$-$R^e$, wherein
$R^1$ and $R^2$ are independently from each other -OH or -O-C(=O)-$R^k$, with $R^k$ being a $C_{1-4}$-alkyl, in particular isopropyl,
$R^3$ and $R^4$ are independently from each other -OH or $-O-(CH_2)_{1-4}-CH_3$,
$R^a$ of $R^5$ is -(C=O)-, -(C=O)-NH-, -NH-(C=O)-, -(C=O)-O-,
$R^b$ of $R^5$ is a five to six membered heterocycle or $C_6$-aryl,
$R^e$ of $R^5$ is -H, $C_{1-2}$-alkyl, -OH, -COOH, $-NH_2$, $-N_3$ or $-SO_2F$,
m is 4 to 8 and the the elements of $R^d$ are independently selected from $-(CH_2)_{1-2}$-O-,
z of $X_z$ is 2 and each $X_z$ is independently from each other $X_z$ -F, -Cl, -Br or -I.

In certain embodiments, one of $R^{5'}$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$ and the other one of $R^5$ and $R^{5''}$, in particular $R^{5''}$, is -OH or both $R^{5'}$ and $R^{5''}$ are independently from each other selected from -O-L-$R^a$-$R^e$, - O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$, in particular
one of $R^5$ and $R^{5''}$, in particular $R^{5'}$, is selected from -O-L-$R^a$-$R^e$, -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$ and the other one of $R^{5'}$ and $R^{5''}$, in particular $R^{5''}$, is -OH, more particularly
both $R^{5'}$ and $R^{5''}$ are independently from each other selected from -O-L-$R^a$-$R^e$, -O-L-$R^a$-$R^b$-$R^e$, -O-L-$R^a$-$R^b$-$R^a$-$R^e$, -O-L-$R^6$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$, wherein
$R^1$ and $R^2$ are independently from each other -OH or -O-C(=O)-$R^k$, with $R^k$ being a $C_{1-4}$-alkyl, in particular isopropyl,
$R^3$ and $R^4$ are independently from each other -OH or $-O-(CH_2)_{1-4}-CH_3$,
$R^a$ of $R^5$ is -(C=O)-, -(C=O)-NH-, -NH-(C=O)-, -(C=O)-O-,
$R^b$ of $R^5$ is a five to six membered heterocycle or $C_6$-aryl,
$R^e$ of $R^5$ is -H, $C_{1-2}$-alkyl, -OH, -COOH, $-NH_2$, $-N_3$ or $-SO_2F$,
m is 4 to 8 and the the elements of $R^d$ are independently selected from $-(CH_2)_{1-2}$-O-,
z of $X_z$ is 2 and each $X_z$ is independently from each other $X_z$ -F, -Cl, -Br or -I.

In certain embodiments, one of $R^{5'}$ and $R^{5''}$, in particular $R^{5'}$, is selected from -O-L-$R^a$-$R^b$-$R^e$, - O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$,$R^e$, -O-$R^d$-$R^e$, -O-L-$R^b$-$R^d$-L-$R^e$, or -O-$R^d$-L-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^b$-$R^e$, -O-L-$R^a$-$R^b$-$R^a$-$R^e$ -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$ -O-L-$R^b$-$R^d$-L-$R^e$, or -O-$R^d$-L-$R^e$, in particular
one of $R^{5'}$ and $R^{5''}$ , in particular $R^{5'}$, is selected from -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or - O-$R^d$-L-$R^e$ and the other one of $R^{5'}$ and $R^{5''}$, in particular $R^{5''}$, is -OH, more particularly
both $R^{5'}$ and $R^{5''}$ are independently from each other selected from -O-L-$R^a$-$R^b$-$R^e$, -O-L-$R^a$-$R^b$-$R^a$-$R^e$, -O-L-$R^e$, -O-L-$R^a$-$R^e$, -O-L-$R^b$-$R^b$-$R^e$, -O-L-$R^b$-$R^e$, -O-L-$R^b$-L-$R^b$-$R^e$, -O-$R^d$-$R^e$, -O-L-$R^b$-$R^d$-L-$R^e$, or -O-$R^d$-L-$R^e$, wherein
$R^1$ and $R^2$ are independently from each other -OH or -O-C(=O)-$R^k$, with $R^k$ being a $C_{1-4}$-alkyl, in particular isopropyl,
$R^3$ and $R^4$ are independently from each other -OH or $-O-(CH_2)_{1-4}-CH_3$,
$R^a$ of $R^5$ is -(C=O)-, -(C=O)-NH-, -NH-(C=O)-, -(C=O)-O-,
$R^b$ of $R^5$ is a five to six membered heterocycle or $C_6$-aryl,
$R^e$ of $R^5$ is -H, $C_{1-2}$-alkyl, -OH, -COOH, $-NH_2$, $-N_3$ or $-SO_2F$,
m is 4 to 8 and the the elements of $R^d$ are independently selected from $-(CH_2)_{1-2}$O-,
z of $X_z$ is 2 and each $X_z$ is independently from each other $X_z$ -F, -Cl, -Br or -I.

In certain embodiments, one of $R^{5'}$ and $R^{5''}$, in particular $R^{5'}$, is selected from -O-L-$R^a$-$R^b$-$R^e$, - O-L-$R^b$-L-$R^b$-$R^e$, -O-$R^d$-$R^e$, -O-L-$R^b$-$R^d$-L-$R^e$, -O-L-$R^a$-$R^b$-$R^a$-$R^e$, -O-L-$R^b$-$R^b$-$R^e$, or -O-$R^d$-L-$R^e$ and the other one of $R^{5'}$ and $R^{5''}$, in particular $R^5$, is -OH or both $R^{5'}$ and $R^{5''}$ are independently from each other selected from -O-L-$R^a$-$R^b$-$R^e$, -O-L-$R^b$-L-$R^b$-$R^e$,

-O-$R^d$-$R^e$, -O-L-$R^b$-$R^d$-L-$R^e$, -O-L-$R^a$$R^b$-$R^a$-$R^e$, -O-L-$R^b$-$R^b$-$R^e$, or -O-$R^d$-L-$R^e$, in particular one of $R^{5'}$ and $R^{5''}$, in particular $R^{5'}$, is selected from -O-L-$R^a$-$R^b$-$R^e$, -O-L-$R^b$-L-$R^b$-$R^e$, -O-$R^d$-$R^e$, -O-L-$R^b$-$R^d$-L-$R^e$, -O-L-$R^a$-$R^b$-$R^a$-$R^e$, -O-L-$R^b$-$R^b$-$R^e$, or -O-$R^d$-L-$R^e$ and the other one of $R^{5'}$ and $R^{5''}$, in particular $R^{5''}$, is -OH, more particularly

both $R^{5'}$ and $R^5$ are independently from each other selected from -O-L-$R^a$-$R^b$-$R^e$, -O-L-$R^b$-L-$R^b$-$R^e$, -O-$R^d$-$R^e$, -O-L-$R^b$-$R^d$-L-$R^e$, -O-L-$R^a$-$R^b$-$R^a$-$R^e$, -O-L-$R^b$-$R^b$-$R^e$, or -O-$R^d$-L-$R^e$, wherein $R^1$ and $R^2$ are independently from each other -OH or -O-C(=O)-$R^k$, with $R^k$ being a $C_{1-4}$-alkyl, in particular isopropyl,

$R^3$ and $R^4$ are independently from each other -OH or -O-$(CH_2)_{1-4}$-$CH_3$,

$R^a$ of $R^5$ is -(C=O)-, -(C=O)-NH-, -NH-(C=O)-, -(C=O)-O-,

$R^b$ of $R^5$ is a five to six membered heterocycle or $C_6$-aryl,

$R^e$ of $R^5$ is -H, $C_{1-2}$-alkyl, -OH, -COOH, -$NH_2$, -$N_3$ or -$SO_2F$,

m is 4 to 8 and the the elements of $R^d$ are independently selected from -$(CH_2)_{1-2}$-O-,

z of $X_z$ is 2 and each $X_z$ is independently from each other $X_z$ -F, -Cl, -Br or -I.

In certain embodiments, one of $R^{5'}$ and $R^{5''}$, in particular $R^{5'}$, is selected from -O-L-$R^a$-$R^b$-$R^e$, - O-L-$R^b$-L-$R^b$-$R^e$, -O-$R^d$-$R^e$, or -O-L-$R^b$-$R^d$-L-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^b$-$R^e$, -O-L-$R^b$-L-$R^b$-$R^e$, -O-$R^d$-$R^e$, or -O-L-$R^b$-$R^d$-L-$R^e$, in particular one of $R^{5'}$ and $R^{5''}$, in particular $R^{5'}$, is selected from -O-L-$R^a$-$R^b$-$R^e$, -O-L-$R^b$-L-$R^b$-$R^e$, -O-$R^d$-$R^e$, or -O-L-$R^b$-$R^d$-L-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^b$-$R^e$, -O-L-$R^b$-L-$R^b$-$R^e$, -O-$R^d$-$R^e$, or -O-L-$R^b$-$R^d$-L-$R^e$, wherein

$R^1$ and $R^2$ are independently from each other -OH or -O-C(=O)-$R^k$, with $R^k$ being a $C_{1-4}$-alkyl, in particular isopropyl,

$R^3$ and $R^4$ are independently from each other -OH or -O-$(CH_2)_{1-4}$-$CH_3$,

$R^a$ of $R^5$ is -(C=O)-, -(C=O)-NH-, -NH-(C=O)-, -(C=O)-O-,

$R^b$ of $R^5$ is a five to six membered heterocycle or $C_6$-aryl,

$R^e$ of $R^5$ is -H, $C_{1-2}$-alkyl, -OH, -COOH, -$NH_2$, -$N_3$ or -$SO_2F$,

m is 4 to 8 and the the elements of $R^d$ are independently selected from -$(CH_2)_{1-2}$O-,

z of $X_z$ is 2 and each $X_z$ is independently from each other $X_z$ -F, -Cl, -Br or -I.

In certain embodiments, one of $R^{5'}$ and $R^{5''}$, in particular $R^{5'}$, is selected from -O-L-$R^a$-$R^e$, -O-L-$R^b$-L-$R^b$-$R^e$, or -O-$R^d$-L-$R^b$-$R^e$ and the other one of $R^{5'}$ and $R^{5''}$, in particular $R^{5''}$, is -OH or both $R^{5'}$ and $R^{5''}$ are independently from each other selected from -O-L-$R^a$-$R^e$, -O-L-$R^b$-L-$R^b$-$R^e$, or -O-$R^d$-L-$R^b$-$R^e$, in particular

one of $R^{5'}$ and $R^{5''}$, in particular $R^{5'}$ is selected from -O-L-$R^a$-$R^e$, -O-L-$R^b$-L-$R^b$-$R^e$, or -O-$R^d$-L-$R^b$-$R^e$ and the other one of $R^{5'}$ and $R^{5''}$, in particular $R^{5''}$, is -OH, more particularly

both $R^{5'}$ and $R^{5''}$ are independently from each other selected from -O-L-$R^a$-$R^e$, -O-L-$R^b$-L-$R^b$-$R^e$, or -O-$R^d$-L-$R^b$-$R^e$, wherein

$R^1$ and $R^2$ are independently from each other -OH or -O-C(=O)-$R^k$, with $R^k$ being a $C_{1-4}$-alkyl, in particular isopropyl,

$R^3$ and $R^4$ are independently from each other -OH or -O-$(CH_2)_{1-4}$-$CH_3$,

$R^a$ of $R^5$ is -(C=O)-, -(C=O)-NH-, -NH-(C=O)-, -(C=O)-O-,

$R^b$ of $R^5$ is a five to six membered heterocycle or $C_6$-aryl,

$R^e$ of $R^5$ is -H, $C_{1-2}$-alkyl, -OH, -COOH, -$NH_2$, -$N_3$ or -$SO_2F$,

m is 4 to 8 and the the elements of $R^d$ are independently selected from -$(CH_2)_{1-2}$-O-,

z of $X_z$ is 2 and each $X_z$ is independently from each other $X_z$ -F, -Cl, -Br or -I.

In certain embodiments, the compound is selected from

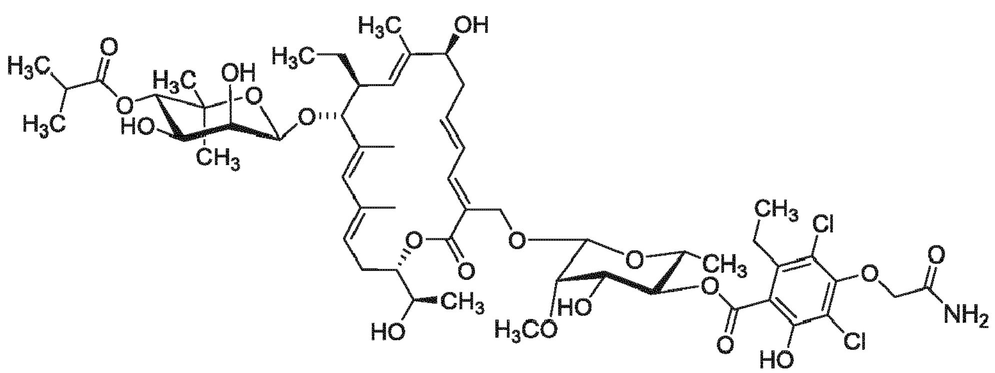

(4a)

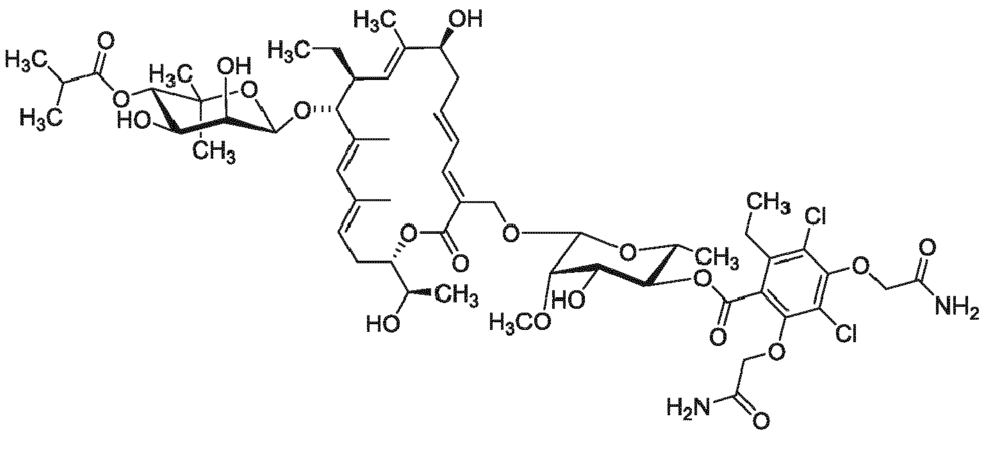

(4b)

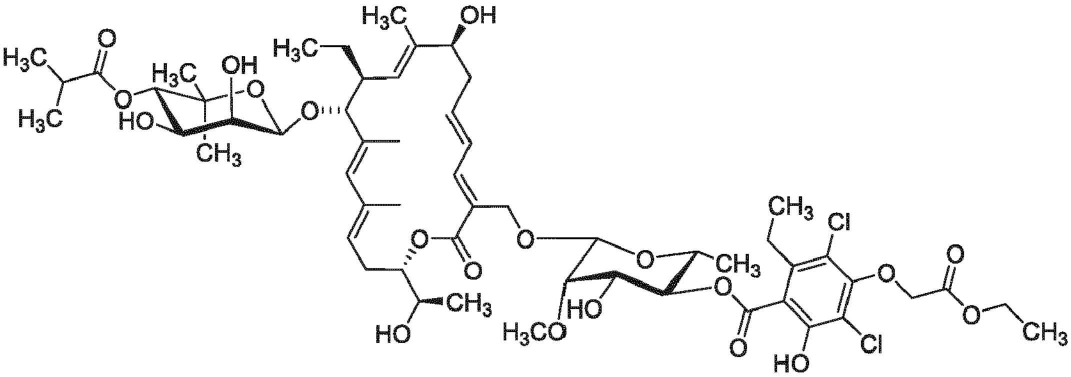

(5a)

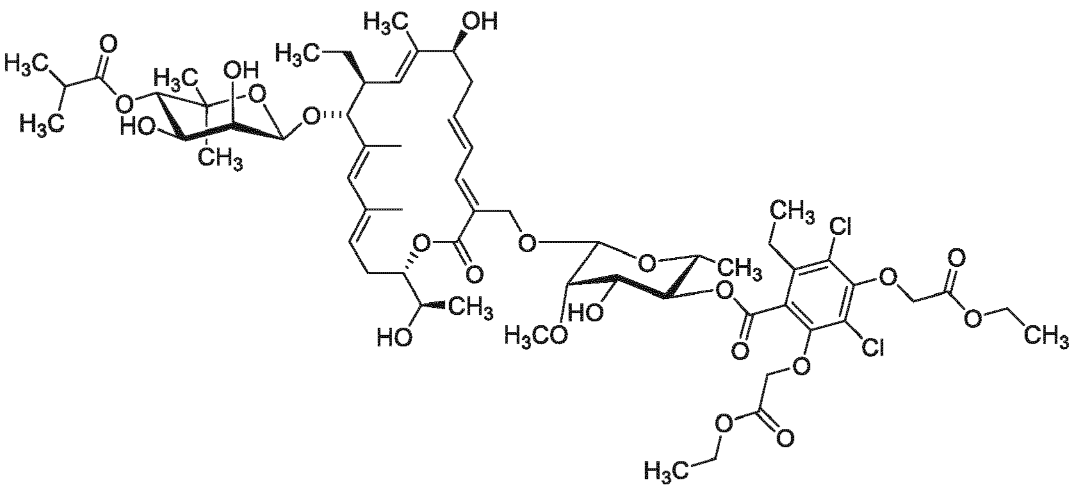

(5b)

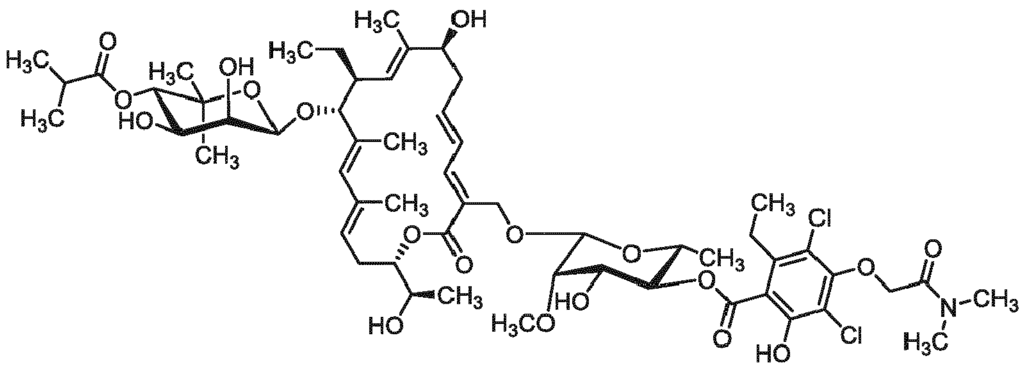

(6a)

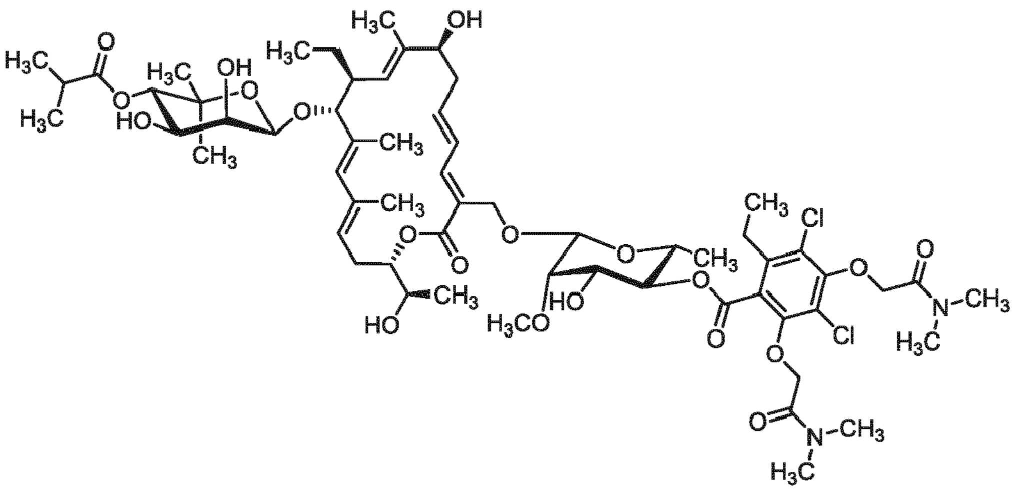

(6b)

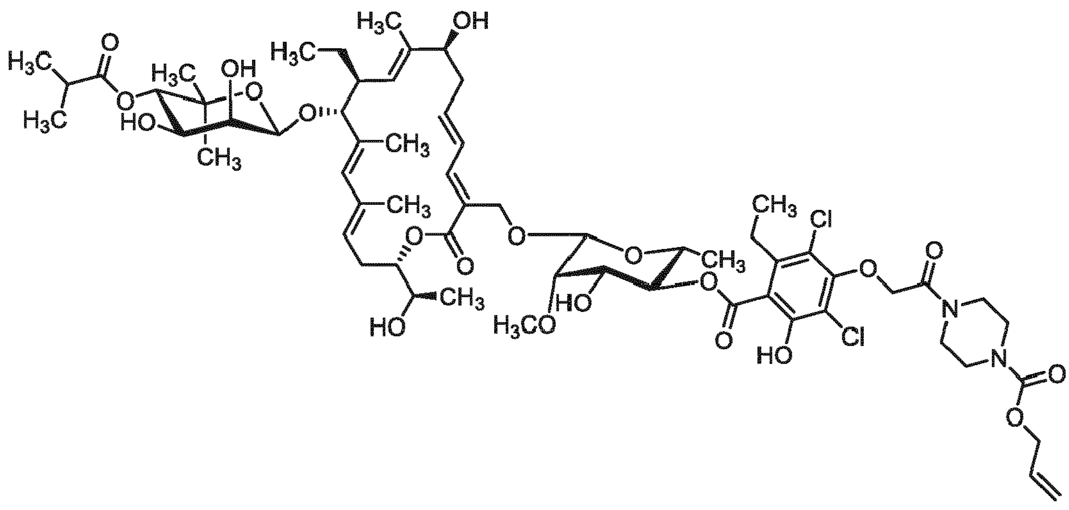

(7a)

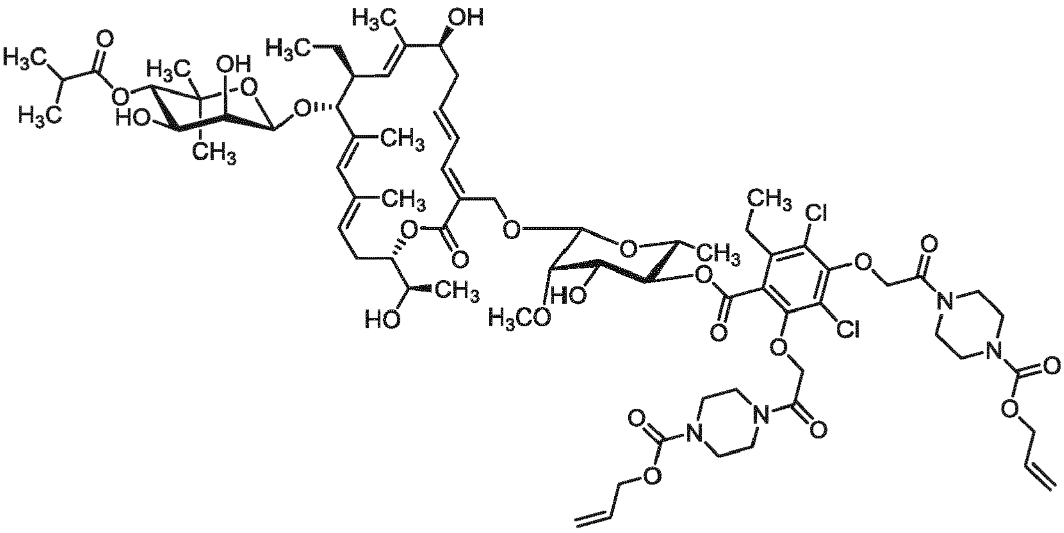

(7b)

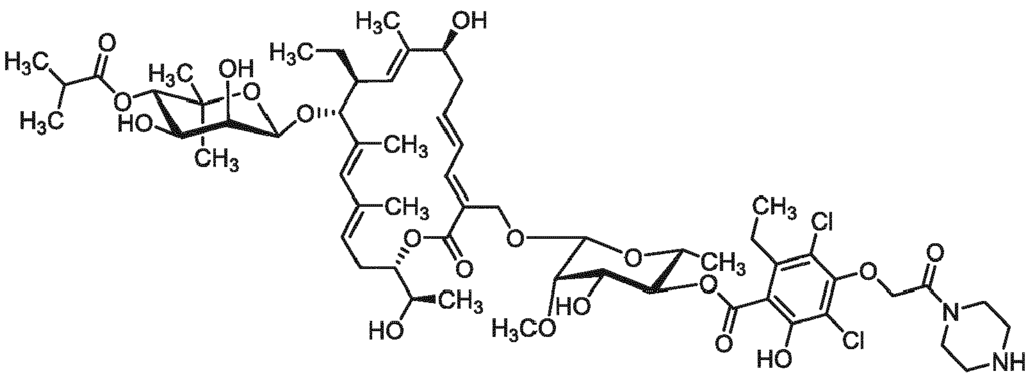

(8)

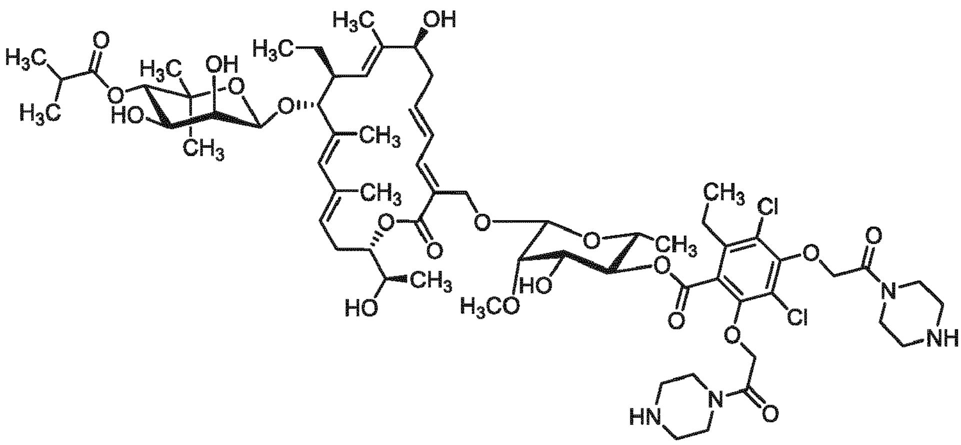

(9)

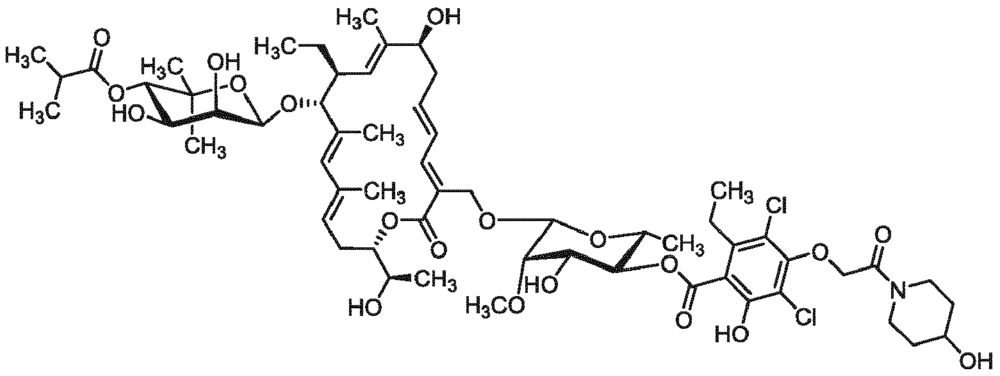

(10a)

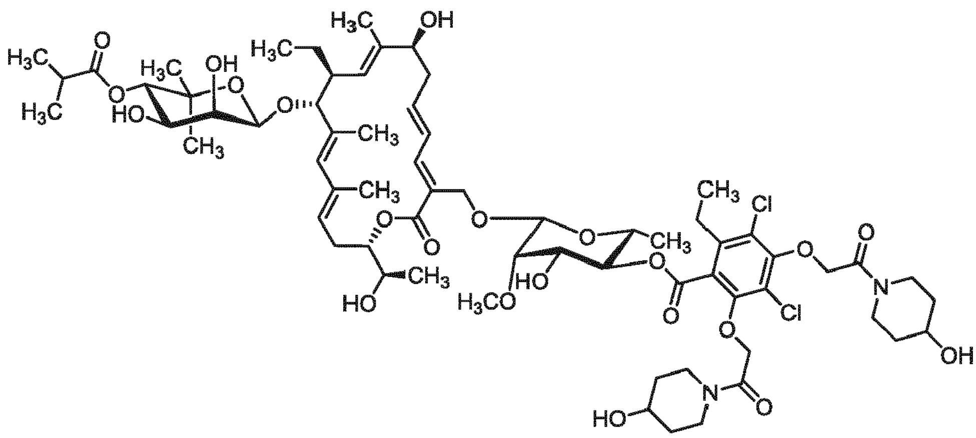

(10b)

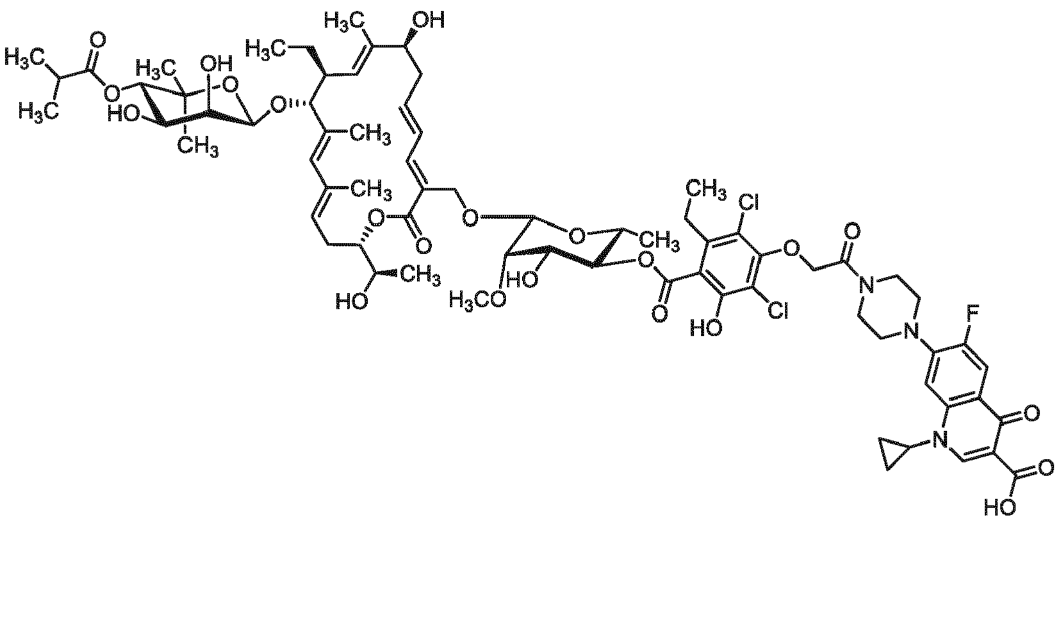

(11a)

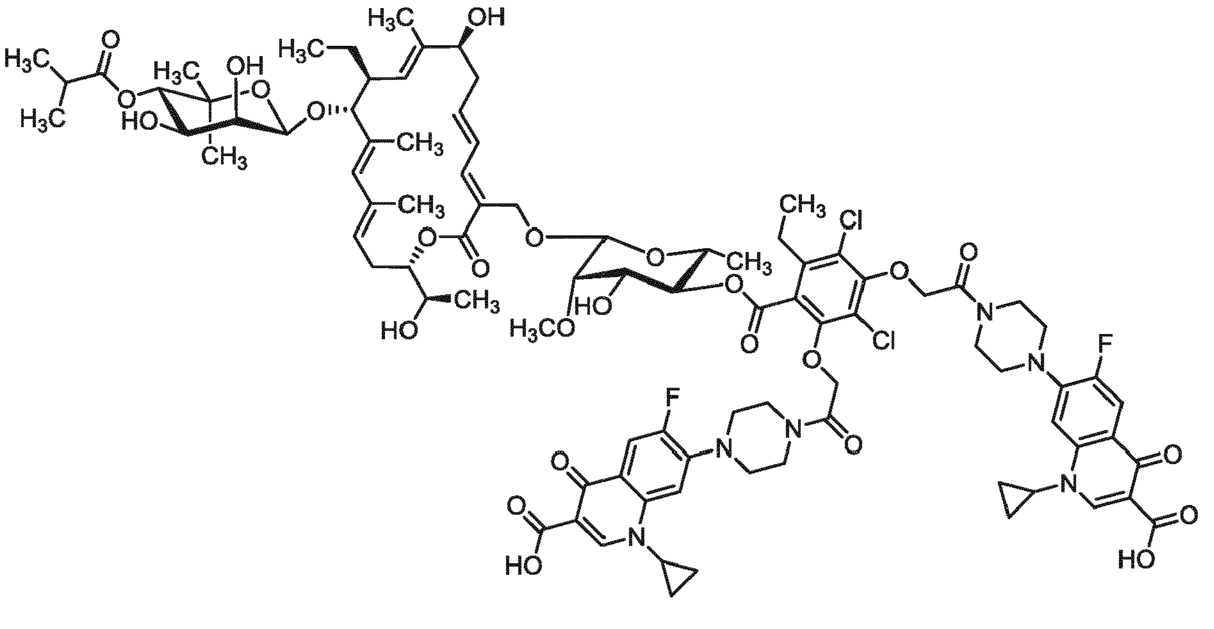

(11b)

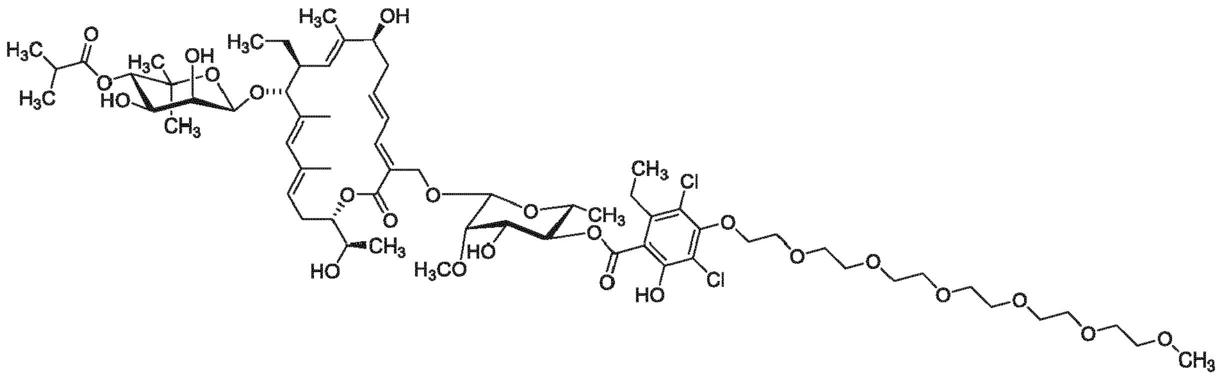

(12a)

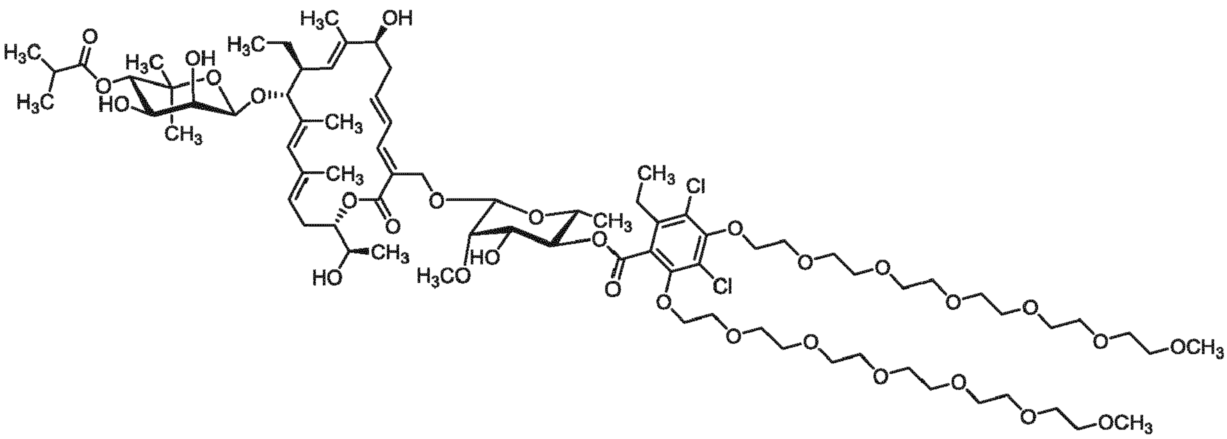

(12b)

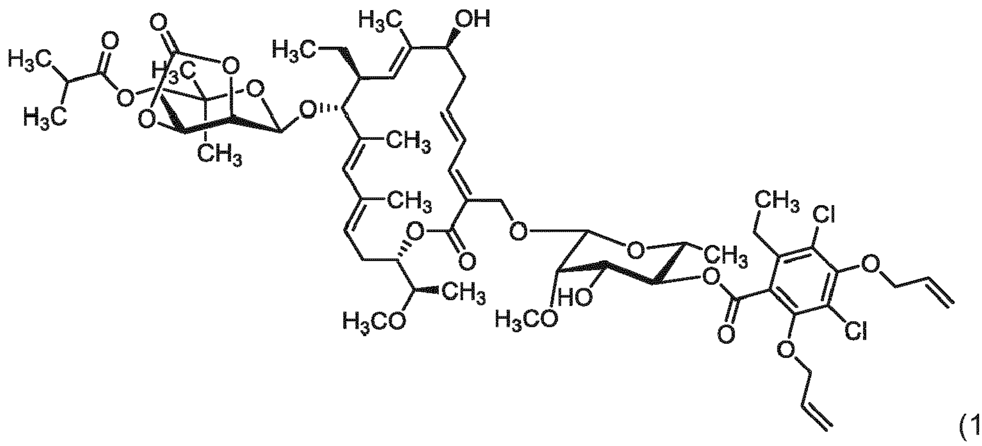

(15a).

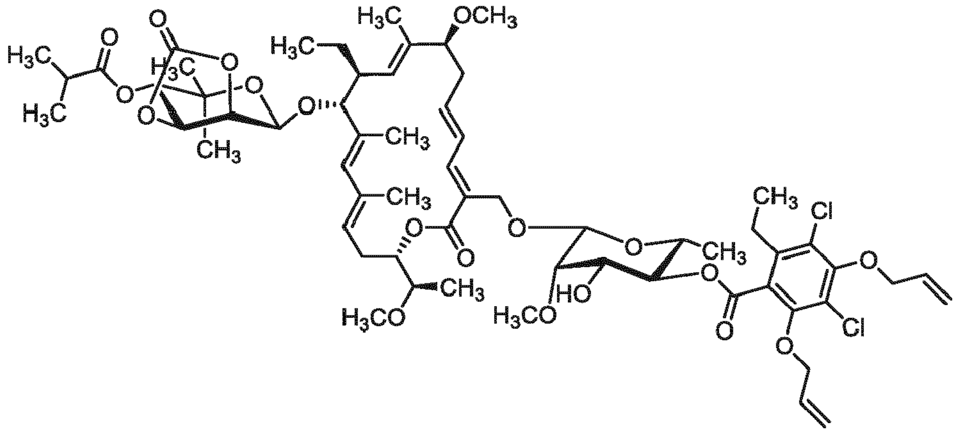

(15b)

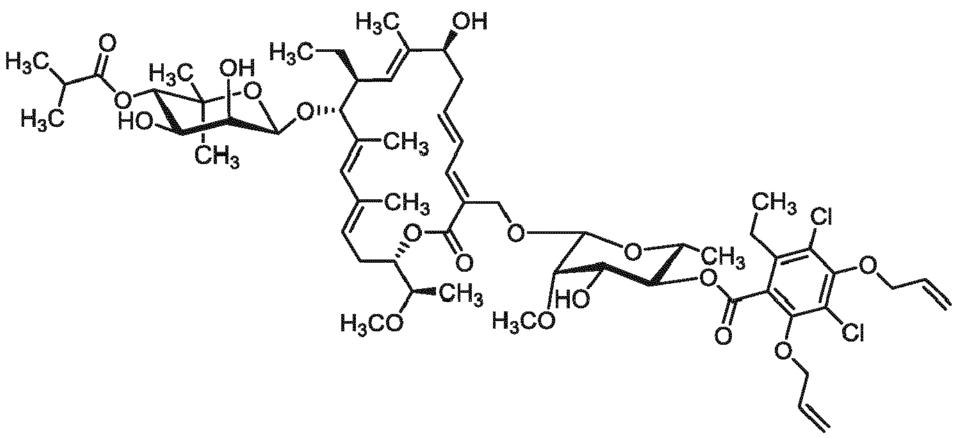

(16)

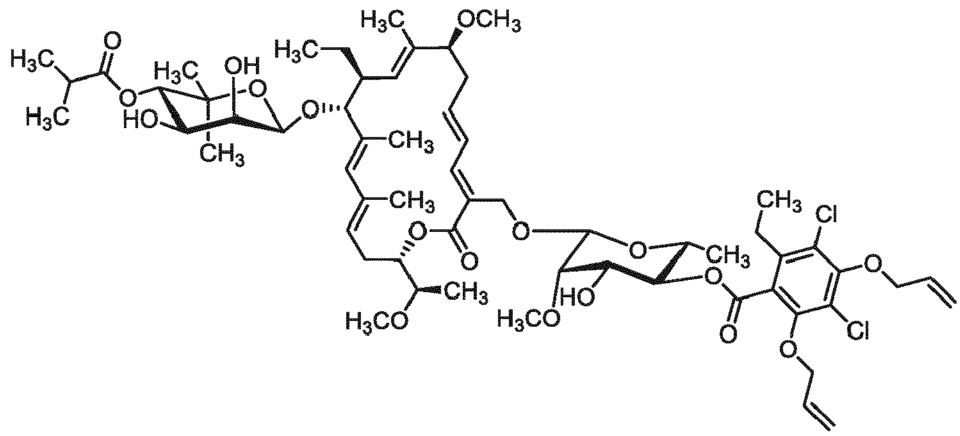

(17)

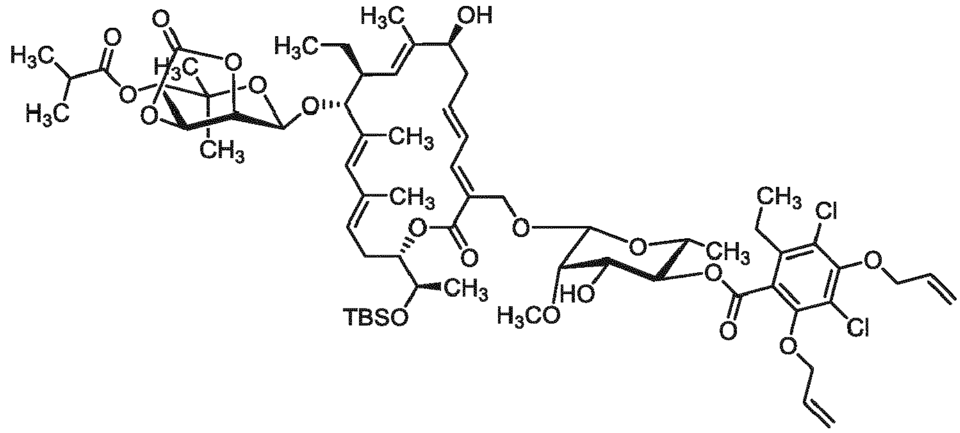

(20)

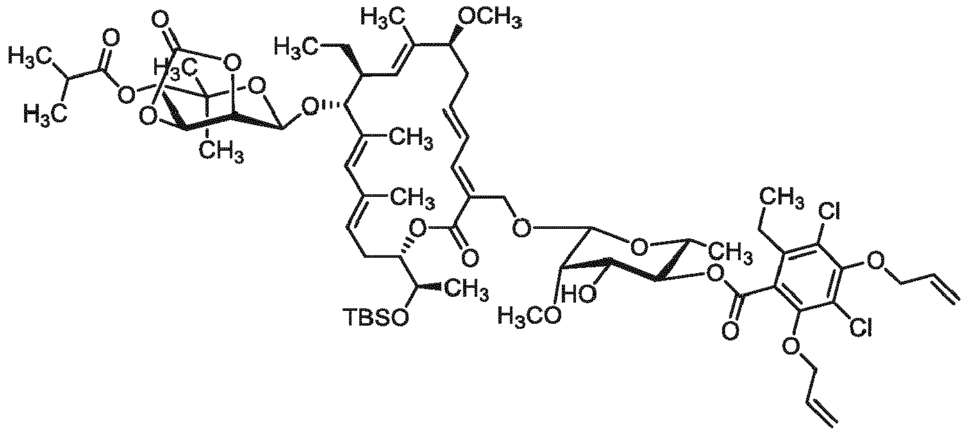

(21a)

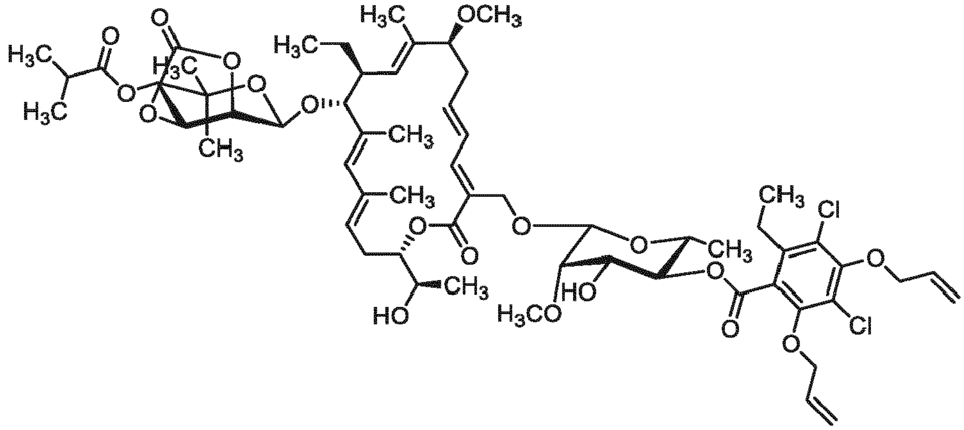

(21b)

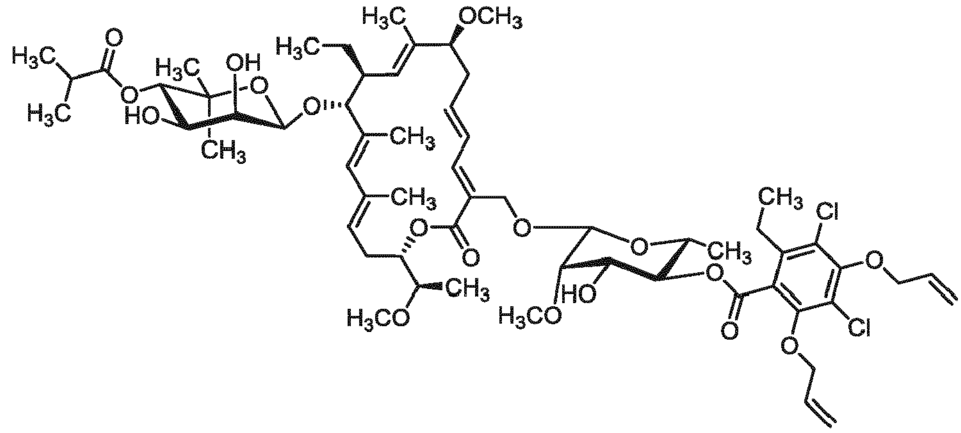

(21c)

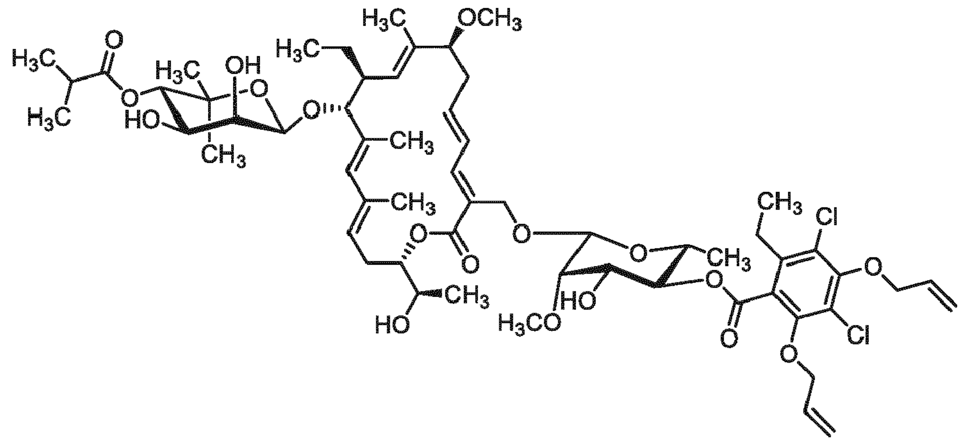

(21d)

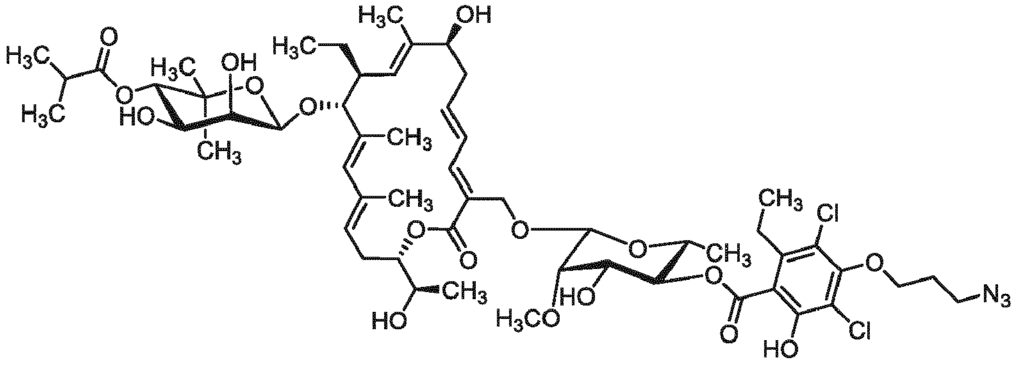

(22a)

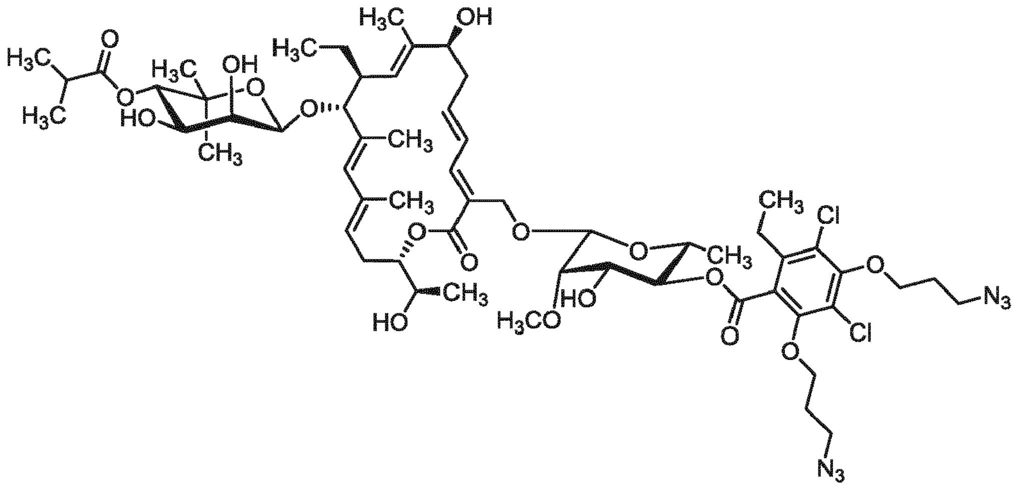

(22b)

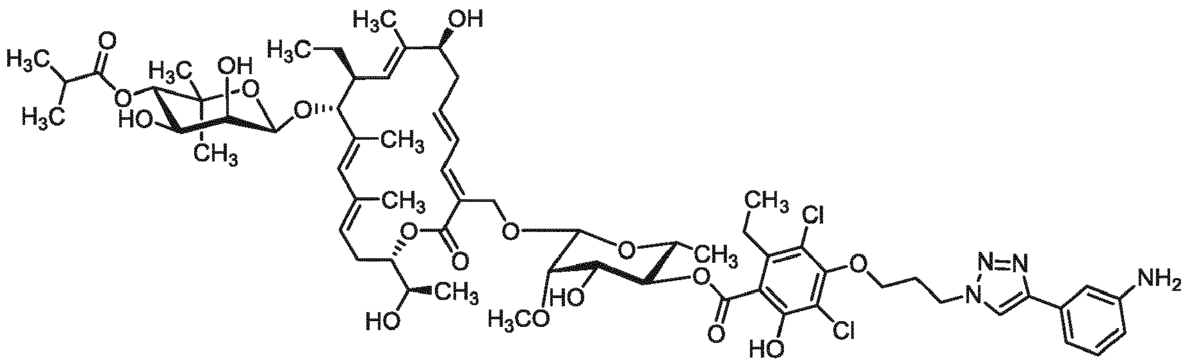

(23a)

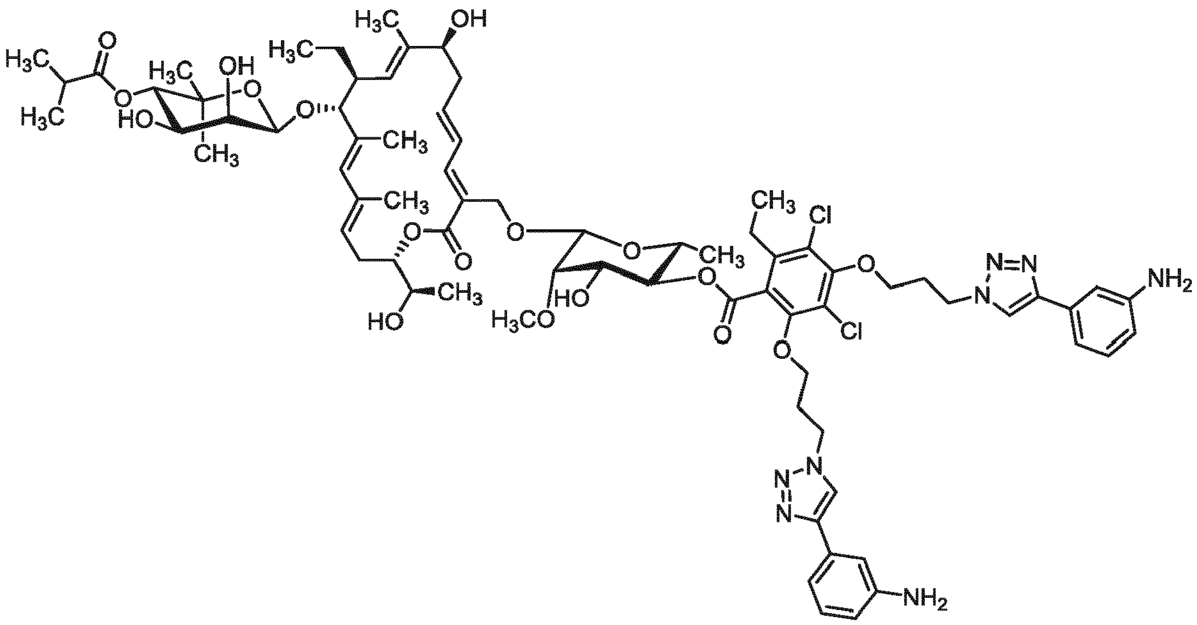

(23b)

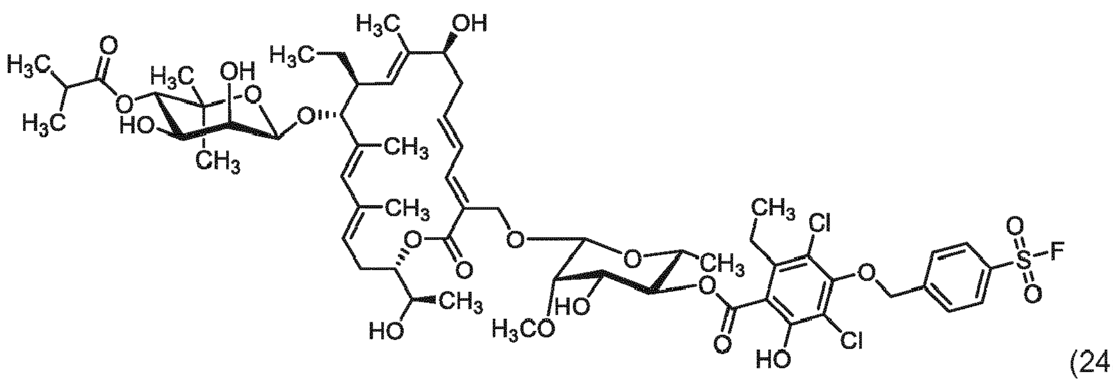

(24a)

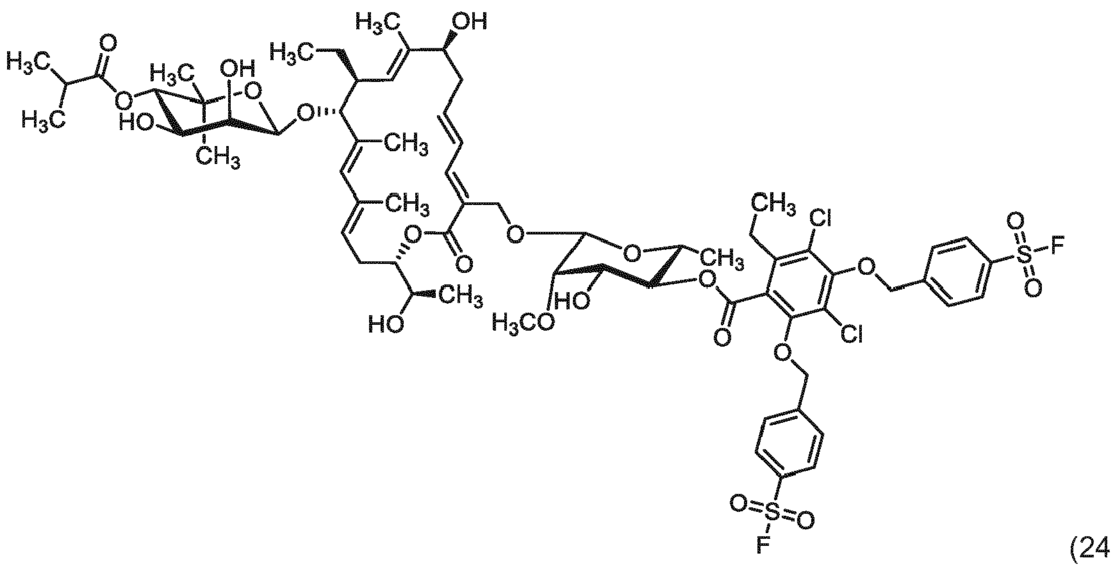

(24b)

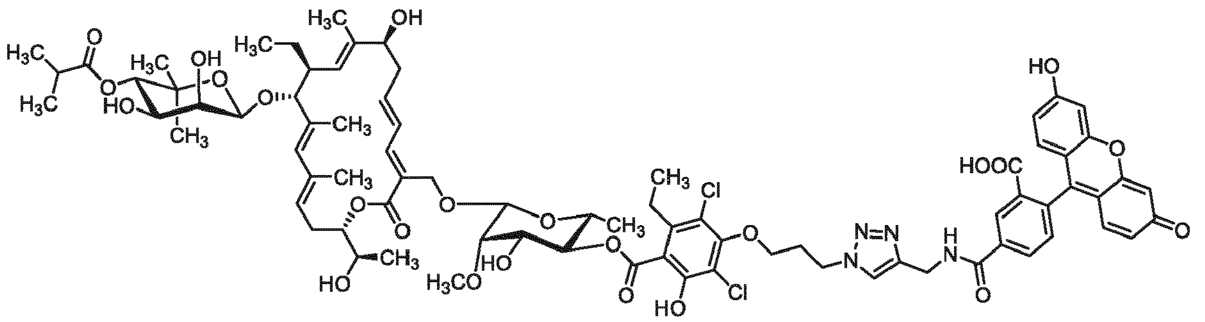

(25)

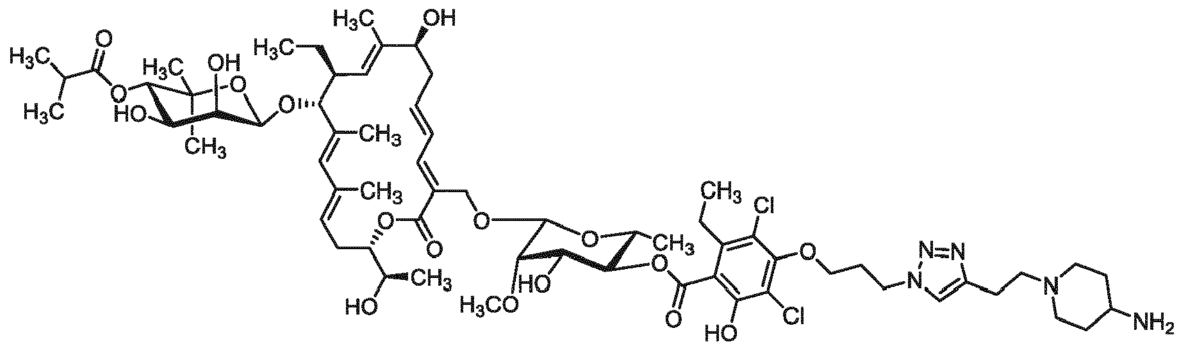

(26a)

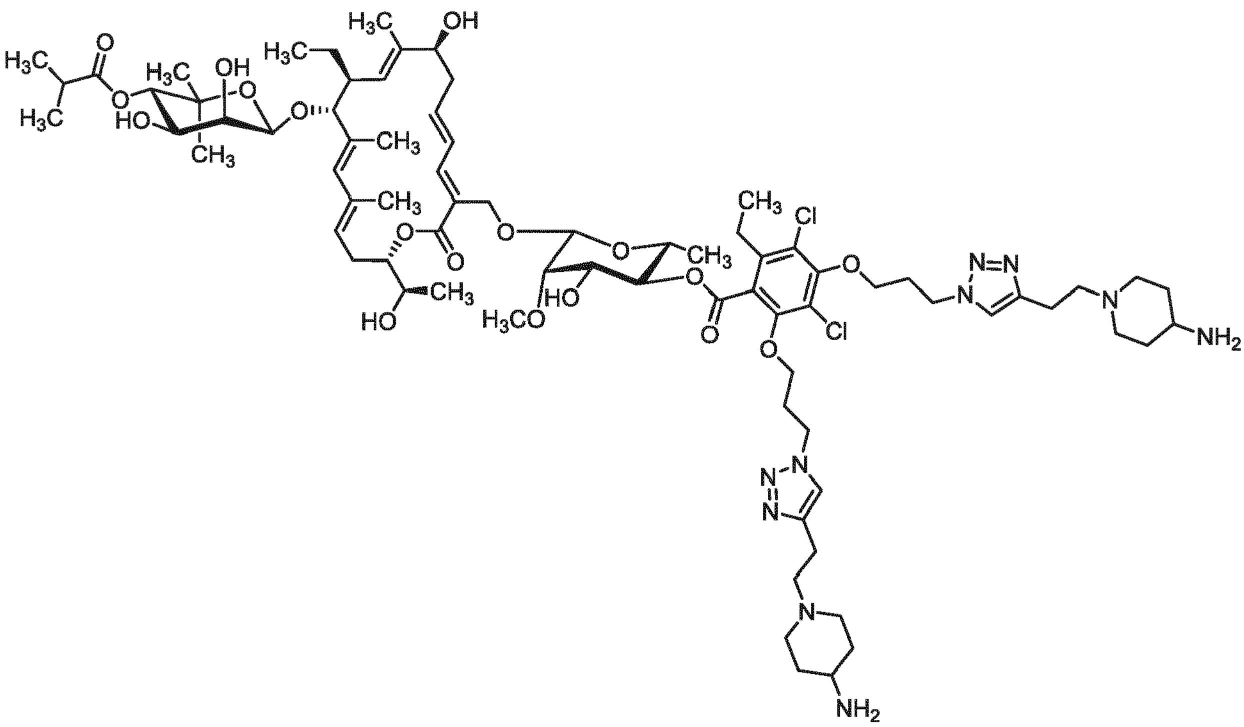

(26b)

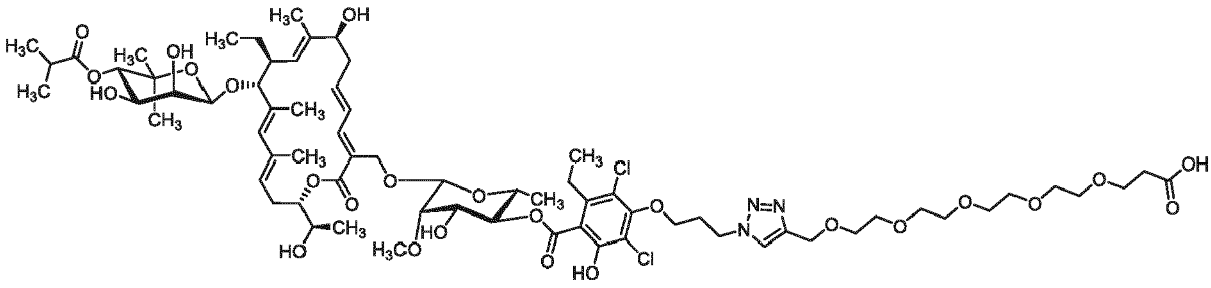

(27)

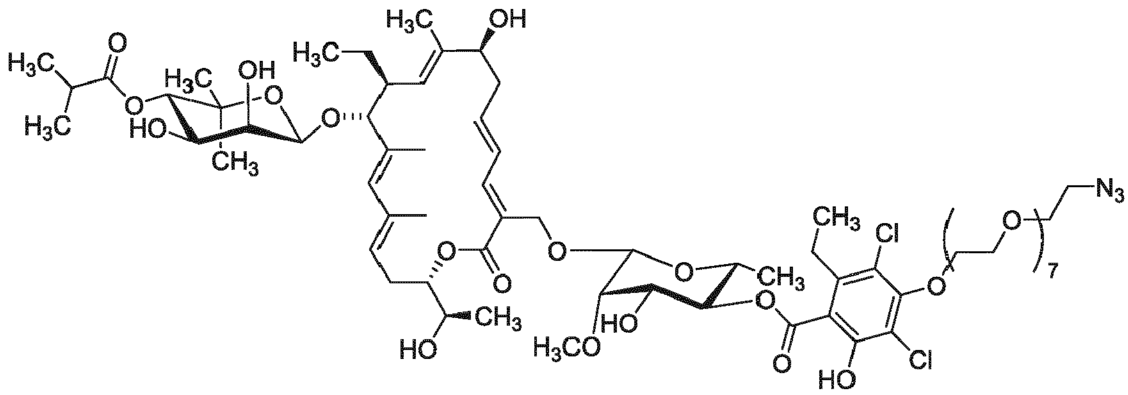

(28)

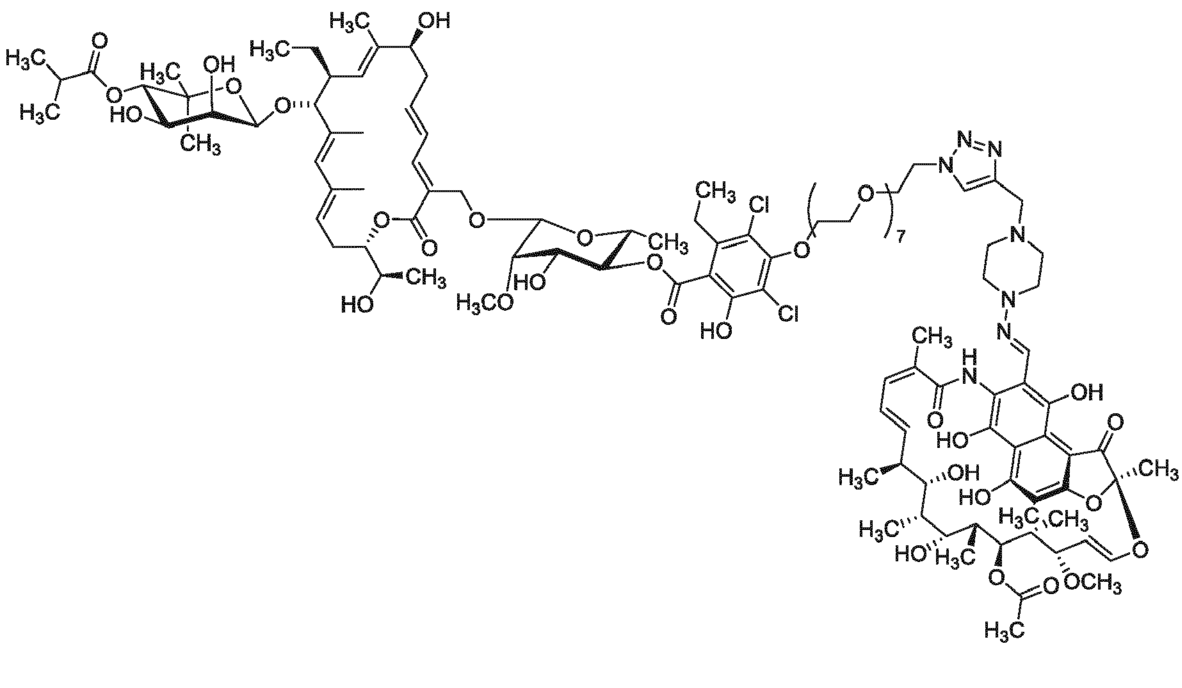

(29).

In certain embodiments, the compound is selected from compound 4a, 4b, 5a, 5b, 6a, 6b, 7a, 7b, 8, 9, 10a, 10b, 11a, 11 b, 12a, 12b, 22a, 22b, 23a, 23b, 24a, 24b, 25, 26a, 26b, 27, 28, 29.

In certain embodiments, the compound is selected from compound 4a, 4b, 5a, 5b, 6a, 6b, 7a, 7b, 8, 9, 10a, 10b, 12a, 12b, 22a, 22b, 23a, 23b, 24a, 24b, 26a, 26b, 27, 28.

In certain embodiments, the compound is selected from compound 4a, 4b, 5a, 5b, 6a, 6b, 7a, 7b, 8, 9, 10a, 10b, 12a, 12b, 23a, 23b, 24a, 24b, 26a, 26b, 27, 28.

In certain embodiments, the compound is selected from compound 7a, 7b, 8, 9, 10a, 10b, 12a, 12b, 23a, 23b, 24a, 24b, 26a, 26b, 27, 28.

In certain embodiments, the compound is selected from compound 7a, 7b, 8, 9, 10a, 10b, 12a, 12b, 23a, 23b, 26a, 26b, 27, 28.

In certain embodiments, the compound is selected from compound 7a, 7b, 8, 9, 10a, 10b, 12a, 12b, 26a, 26b, 27, 28.

In certain embodiments, the compound is selected from compound 10a, 10b, 12a, 12b, 26a, 26b,27.

In certain embodiments, the compound is selected from compound 10a, 10b, 12b, 26b, 27.

In certain embodiments, the compound is selected from 5a, 5b, 11a, 11b, 26a, 26b, 29.

In certain embodiments, the compound is selected from 5a, 5b, 11a, 26b, 29.

According to a second aspect of the invention the compounds of the first aspect of the invention are provided for use in a method of treatment of disease.

According to a third aspect of the invention a compound of the first aspect of the invention is provided for use in a method of treatment of infections, in particular bacterial infections, more particularly infections caused by

- *Clostridioides,* in particular *Clostridium difficile* or *Clostridium perfringens,*
- *Mycobacteria,* in particular Mycobacterium tuberculosis,
- *Staphylococci,* in particular *Staphylococcus aureus,*
- *Enterococci,* in particular *Enterococcus faecium,*
- *Escherichia,* in particular *Escherichia coli,* and
- *Bacilli,* and

for use in a method of treatment of bacterial infections caused by drug resistant bacteria, in particular

- resistant *Mycobacterium tuberculosis,*
- resistant *Staphylococcus* species, in particular methicillin-resistant *Staphylococcus aureus,*
- resistant *Enterococci,* in particular vancomycin-resistant *Enterococci.*

In certain embodiments, the compounds of the first aspect of the invention are used in a method of treatment of infections, in particular bacterial infections, more particularly infections caused by

- *Clostridioides,* in particular *Clostridium difficile* or *Clostridium perfringens,*
- *Mycobacteria,* in particular Mycobacterium tuberculosis,
- *Staphylococci,* in particular *Staphylococcus aureus,*
- *Enterococci,* in particular *Enterococcus faecium,*
- *Escherichia,* in particular *Escherichia coli,* and
- *Bacilli.*

In certain embodiments, the compounds of the compounds of the first aspect of the invention are used in a method of treatment of bacterial infections caused by drug resistant bacteria, in particular

- resistant *Mycobacterium tuberculosis,*
- resistant *Staphylococcus* species, in particular methicillin-resistant *Staphylococcus aureus,*
- resistant *Enterococci,* in particular vancomycin-resistant *Enterococci.*

In certain embodiments, the bacteria are $gram^+$ or $gram^-$.

Similarly, a dosage form for the prevention or treatment of bacterial infections is provided, comprising a compound according to one of the above aspects of the invention. Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present. The dosage form may comprise one fidaxomicin derivative according to the invention or several different fidaxomicin derivatives according to the invention ("cocktail"). If the dosage form is a cocktail, typically 2 to 10, in particular 2 to 4 different fidaxomicin derivatives are present.

Wherever alternatives for single separable features such as, for example, a functional group are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

## Examples

*Synthesis of compounds:*

*General Experimental*

Unless otherwise stated, all chemicals were of reagent grade and purchased from *Sigma-Aldrich, Merck, Fluorochem, Honeywell,* or *Thommen Furler AG.* Reactions were carried out under protecting gas ($N_2$ or Ar) in oven-dried (120°C) glass equipment and monitored for completion by TLC or UHPLC-MS (ESI). Solvents for reactions were of p.a. grade. Evaporation of solvents *in vacuo* was carried out on a rotary evaporator at 40 °C bath temperature and appropriate pressure. Thin layer chromatography (TLC): *Merck* TLC plates silica gel 60 on aluminum with the indicated solvent system; the spots were visualized by UV light (254 nm) and $KMnO_4$ stain. **Ultra high-performance liquid chromatography coupled to mass spectrometry** (UHPLC-MS): *Ultimate 3000 LC* instrument (*Thermo Fisher Scientific*) coupled to a triple quadrupole *Quantum Ultra EMR* MS (*Thermo Fisher Scientific*) using a reversed-phase column (*Kinetex®* EVO C18; 1.7 μm; 100 Å, 50 x 2.1 mm; *Phenomenex*). The LC was equipped with a *HPG-3400RS* pump, a *WPS-3000TRS* autosampler, a *TCC-3000RS* column oven and a *Vanquish DAD* detector (all *Thermo Fisher Scientific*). The following solvents were applied: $H_2O$+0.1 % HCOOH (A), MeCN+0.1 % HCOOH (B). The MS was equipped with an H-ESI II ion source. The source temperature was 250 °C, the capillary temperature 270 °C and capillary voltage 3500 V, and datasets were acquired at resolution 0.7 on Q3 in centroid mode. **High-performance liquid chromatography** (HPLC): *Prominence* modular HPLC instrument (*Shimadzu*) coupled to a *SPD-20A* UV/Vis detector (*Shimadzu*) using a reversed-phase column (*Gemini-NX* C18, 3 μm,10Å, 150 mm x 4.6 mm; *Phenomenex*) for analytical HPLC and a reversed-phase column (*Gemini NX C18,* 5 μm, 110 Å, 250 mm x 21.2 mm; *Phenomenex Synergi Hydro-RP,* 10 μ, 80 Å, 250 mm x 21.2 mm; *Phenomenex*) for preparative HPLC. The LC was equipped with a *CBM-20A* system controller, *LC-20A* solvent delivery unit, *SIL-20A* auto-sampler, *CTO-20A* column oven, and *DGU-20A* online degassing unit (all *Shimadzu*). The following solvents were used: $H_2O$+0.1 % HCOOH (A), MeCN + 0.1 % HCOOH (B). **Specific optical rotation** $[\alpha]_D^T$: *Jasco P-2000 Polarimeter,* measured at the indicated temperature *T.* **Infrared spectra** (IR): *SpectrumTwo FT-IR Spectrometer* (*Perkin-Elmer*) equipped with a *Specac Golden Gate™ ATR* (attenuated total reflection) accessory; applied as neat samples or as films; $1/\lambda$ in $cm^{-1}$. **Nuclear magnetic resonance spectra** (NMR): $^1H$ NMR spectra were recorded in $CDCl_3$, $CD_2Cl_2$, acetone-$d_6$ or DMSO-$d_6$ on the instruments *Bruker AV-600* (600 MHz), *Bruker AV-500* (500 MHz) or *AV-400* (400 MHz); $\delta$ in ppm relative to solvent signals ($\delta$ = 7.26 ppm for $CDCl_3$, 5.32 ppm for $CD_2Cl_2$, 2.05 ppm for acetone-$d_6$ and 2.50 for DMSO-$d_6$),[1] *J* is given in Hz. $^{13}C$ NMR spectra were recorded in in $CDCl_3$, $CD_2Cl_2$, acetone-$d_6$ or DMSO-$d_6$ on the instruments *Bruker AV-600* (150 MHz), *Bruker AV-500* (125 MHz) or *AV-400* (100 MHz); $\delta$ in ppm relative to solvent signals ($\delta$ = 77.16 ppm for $CDCl_3$, 53.84 ppm for $CD_2Cl_2$, 29.84 ppm for acetone-$d_6$ and 39.52 for DMSO-$d_6$);[1] multiplicities from DEPT-135 experiments. **High-resolution electrospray ionization mass spectra** (HRMS): *QExactive* (*Thermo Fisher Scientific*) with a heated ESI source connected to a *Dionex Ultimate 3000* UHPLC system. Samples dissolved in MeOH at ca. 50 μg $mL^{-1}$; injection of 1 μL on-flow with an auto-sampler (mobile phase: MeOH+0.1 % HCOOH or MeCN/$H_2O$ 2:8 + 0.1 % HCOOH; flow rate: 120 μL/min); ion source parameters: spray voltage 3.0 kV, capillary temperature 320°, sheath gas rate: 5 l min-1, s-lens RF level 55.0; full scan MS in alternating (+)/(-)-ESI mode; mass ranges 80-1200, 133-2000, or 200-3000 amu; resolution (full width half-maximum) 70'000; automatic gain control (AGC) target $3.00\text{-}10^6$; maximum allowed ion transfer time (IT) 30 ms; mass calibration <2 ppm accuracy for m/z 130.06619-1621.96509 in (+)-ESI and for *m/z* 265.14790-1779.96528 in (-)-ESI with *Pierce®* ESI calibration solutions (*Thermo Fisher Scientific*); lock masses: ubiquitous erucamide (*m/z* 338.34174, (+)-ESI) and palmitic acid (m/z 255.23295, (-)-ESI).

*Compounds (4a) and (4b)*

In a flame-dried flask under argon atmosphere, fidaxomicin (100 mg, 0.0945 mmol, 1.0 eq.) and $K_2CO_3$ (52.2 mg, 0.378 mmol, 4.0 eq.) were dissolved in dry DMF (1.2 mL). Iodoacetamide (22.7 mg, 0.123 mmol, 1.3 eq.) was added and the yellow reaction mixture was stirred at 45 °C under argon atmosphere for 5 h. The reaction mixture was diluted with EtOAc (2.0 mL) and quenched with sat. aq. $NH_4Cl$-solution (3.0 mL). The layers were separated and the aqueous layer was extracted with EtOAc (3x). The combined organic phases were washed with brine (3x), dried over $MgSO_4$ and the solvent was evaporated to afford a mixture of mono-and disubstituted fidaxomicin acetamide. The two compounds 4a and 4b were separated by RP-HPLC (*Gemini NX* C18, 10 μ, 110 Å, 250 mm x 21.2 mm; solvent A: $H_2O$+0.1 % FA, solvent B: MeCN+0.1 % FA; 20 mL/min; 47 % B for 50 min) to afford 4a ($t_R$=16.25 min, 24.0 mg, 0.022 mmol, 23%) and

4b ($t_R$=9.50 min, 6.0 mg, 0.005 mmol, 5%) after lyophilization.
4a: HRMS ESI(-) (MeOH) calculated for $[M-H]^-$: 1112.43941, found: 1112.44043.
4b: HRMS ESI(+) (MeOH) calculated for $[M+Na]^+$: 1193.45737, found: 1193.45811.

*Compounds (5a) and (5b)*

A flame-dried flask was charged with fidaxomicin (20.0 mg, 0.0189 mmol, 1.0 eq.) and $K_2CO_3$ (10.4 mg, 0.0756 mmol, 4.0 eq.). The solids were dissolved in dry DMF (0.8 mL) and ethyliodoacetate (3.0 μL, 5.30 mg, 0.0246 mmol, 1.3 eq.) was added. The slightly yellow reaction mixture was stirred at 45 °C under argon atmosphere for 6.5 h. The reaction mixture was then diluted with EtOAc (1.0 mL) and quenched with sat. aq. $NH_4Cl$ solution (1.0 mL). The layers were separated and the aqueous was extracted with EtOAc (3x). The combined organic phases were washed with brine (2x) dried over $MgSO_4$ and the solvent was evaporated to afford fidaxomicin ethyl acetate 5a and 5b as a mixture. The compounds were separated by silica-gel column chromatography (40% acetone/pentane) to afford 5a (6.20 mg, 0.005 mmol, 29%) and 5b (7.80 mg, 0.006 mmol, 34%) as colourless solids.
5a: HRMS ESI(+) (MeOH) calculated for $[M+Na]^+$: 1165.45122, found: 1165.44810.
5b: HRMS ESI(+) (MeOH) calculated for $[M+NH_4]^+$: 1246.53261, found: 1246.53412.

*Compounds (6a) and (6b)*

To an oven dried flask charged with fidaxomicin (20.0 mg, 0.0189 mmol, 1.0 eq.), $K_2CO_3$ (10.4 mg, 0.0756 mmol, 4.0 eq.) was added under argon atmosphere. The solids were dissolved in DMF (0.8 mL) and 2-iodo-*N*,*N*-dimethylacetamide (4.5 μL, 8.05 mg, 0.0378 mmol, 2.0 eq.) was added. The dark brown mixture was stirred at 45 °C for 21.5 h.

The reaction mixture was diluted with EtOAc (1.0 mL) and quenched with sat. aq. $NH_4Cl$ solution (1.0 mL). The phases were separated and the aqueous phase was extracted with EtOAc (3x). The combined organic layers were washed with brine (2x), dried over $MgSO_4$ and the solvent was evaporated under reduced pressure. A mixture of mono- and disubstituted fidaxomicin-*N*,*N*-dimethylacetamide 6a and 6b was obtained. The two compounds were separated by RP-HPLC (*Gemini NX* C18*,* 10 μ, 110Å, 250 mm x 21.2 mm; solvent A: $H_2O$+0.1 % FA, solvent B: MeCN+0.1 % FA; 20 mL/min; 47 % B for 50 min) to afford, after lyophilization, 6a ($t_R$=23.83 min, 9.50 mg, 0.008 mmol, 44%) and 6b ($t_R$=16.88 min, 8.5 mg, 0.007 mmol, 37%) as colourless solids.
6a: HRMS ESI(-) (MeOH) calculated for $[M-H]^-$: 1140.47071, found: 1140.47231.
6b: HRMS ESI(+) (MeOH) calculated for $[M+Na]^+$: 1249.51997, found: 1249.51806.

*Allyl 4-(2-chloroacetyl)piperazine-1-carboxylate*

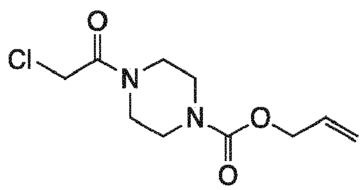

In a flame-dried flask, a solution of chloroacetyl chloride (58.5 μL, 82.9 mg, 0.734 mmol, 1.0 eq.) in dry THF (590 μL) was added to a stirred solution of alloc-protected piperazine (125 mg, 0.734 mmol, 1.0 eq.) and freshly distilled diisopropylethyl amine (146 μL, 114 mg, 0.881 mmol, 1.2 eq.) in THF (2.3 mL) at 0 °C. Upon addition of chloroacetyl chloride, the clear solution turned pink and a colourless solid precipitated. The mixture was stirred for 1 h at 0 °C and was then allowed to warm to rt and stirred for another 2.5 h. The solvent was evaporated and the residue was dissolved in chloroform (5.0 mL). The organic phase was washed with water (3x) and the combined aqueous layers were extracted with chloroform. The combined organic phases were dried over $MgSO_4$ and concentrated under reduced pressure.

The crude product was purified by column chromatography (50% EtOAc/pentane) to afford the desired product as a colorless oil (130 mg, 0.527 mmol, 72%)
HRMS ESI(+) (MeOH) calculated for $[M+H]^+$: 247.08440, found: 247.08442.

*Allyl 4-(2-iodoacetyl)piperazine-1-carboxylate*

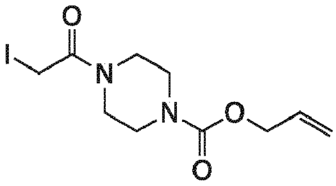

In a flame-dried flask equipped with a reflux condenser, allyl 4-(2-chloroacetyl)piperazine-1-carboxylate (80.0 mg, 0.324 mmol, 1.0 eq.) was dissolved in dry MeCN (1.0 mL) and NaI (219 mg, 1.46 mmol, 4.5 eq.) was added. The mixture immediately turned cloudy yellow and was stirred for 3 h at 60 °C under argon atmosphere. Then water (3.0 mL) was added to the reaction mixture, and MeCN was removed under reduced pressure. EtOAc (3.0 mL) was added and the phases were separated. The aqueous was extracted with EtOAc (3x). The combined yellow organic layers were dried over $MgSO_4$. After filtration of the drying agent, the solvent was evaporated under reduced pressure. The crude product was purified by column chromatography (50% EtOAc/pentane) to afford the desired product (83.0 mg, 0.246 mmol, 76%) as a yellow oil
HRMS ESI(+) (MeOH) calculated for $[M+H]^+$: 339.02001, found: 339.01996.

*Compounds (7a) and (7b)*

A flame-dried flask was charged with fidaxomicin (100 mg, 0.0945 mmol, 1.00 eq.) and $K_2CO_3$ (52.2 mg, 0.378 mmol, 4.0 eq.). The solids were dissolved in dry DMF (4.0 mL) and allyl 4-(2-iodoacetyl)piperazine-1-carboxylate (47.9 mg, 0.142 mmol, 1.5 eq.) was added. The brown reaction mixture was stirred at 45 °C under argon atmosphere for 7.5 h. The reaction mixture was then diluted with EtOAc (4.0 mL) and quenched with sat. aq. $NH_4Cl$ solution (4.0 mL). The layers were separated and the aqueous phase was extracted with EtOAc (3x). The combined organic phases were dried over $MgSO_4$. After filtration of the drying agent, the solvent was evaporated to afford mono-and disubstituted products 7a and 7b as a mixture. The two compounds were separated by RP-HPLC *[Gemini NX* C18, 10 μ, 110 Å, 250 mm x 21.2 mm, solvent A: $H_2O$+0.1 % FA, solvent B: MeCN+0.1 % FA; 20 mL/min; LC time program (min - % B): 0.0 - 45 %, 3.0 - 45 %, 55.0 - 55 %, 46.0 - 100 %] to afford, after lyophilization, 7a ($t_R$=35.6 min, 32.3 mg, 0.025 mmol, 27%) and 7b ($t_R$=38.0 min, 42.8 mg, 0.029 mmol, 31%) as colourless solids.
7a: HRMS ESI(+) (MeOH) calculated for $[M+Na]^+$: 1289.51488, found: 1289.51544.
7b: HRMS ESI(+) (MeOH) calculated for $[M+Na]^+$: 1499.61533, found: 1499.61505.

*Compound (8)*

In a flame-dried flask under argon atmosphere, mono-substituted alloc-piperazine-fidaxomicin (20 mg, 0.0158 mmol, 1.0 eq.) was dissolved in dry DCM (1.5 mL) and 1,3-dimethylbarbituric acid (5.92 mg, 0.0379 mmol, 2.4 eq.) was added. The reaction mixture was cooled to 0 °C and tetrakis(triphenylphosphine)palladium (0.92 mg, 0.79 μmol, 0.05 eq.) was added. The mixture was stirred at 0 °C for 30 min. After completion of the reaction, the mixture was diluted with DCM (2.0 mL) and quenched with $H_2O$ (3.0 mL). The phases were separated and the aqueous phase was extracted with DCM. The combined organic layers were dried over $MgSO_4$ and the solvent was removed under reduced pressure. The crude product was purified by column chromatography (10-20% MeOH/DCM) to afford the desired amine (9.50 mg, 0.008 mmol, 51%) as a colourless solid.
8: HRMS ESI(+) (MeOH) calculated for $[M+H]^+$: 1183.51181, found: 1183.51223.

*Compound (9)*

In a flame-dried flask, disubstituted alloc-piperazine-fidaxomicin (15.4 mg, 0.0104 mmol, 1.0 eq.) was dissolved in dry DCM (1.0 mL) and 1,3-dimethylbarbituric acid (3.9 mg, 0.025 mmol, 2.4 eq.) was added. The reaction mixture was cooled to 0 °C and tetrakis(triphenylphosphine)palladium (0.607 mg, 0. 52 μmol, 0.05 eq.) was added. The mixture was stirred at 0 °C for 1 h. DCM (1.0 mL) and water (2.0 mL) were added. The phases were separated and the aqueous phase was extracted with DCM (3x). The desired product (9.5 mg crude) was obtained after lyophilization of the aquous phase.
9: HRMS ESI(+) (MeOH) calculated for $[M+H]^+$: 1309.59112, found: 1309.59286; calculated for $[M+2H]^{2+}$: 655.29920, found: 655.29992.

*2-Iodo-1-(4-hydroxycyclohexyl)ethan-1-one*

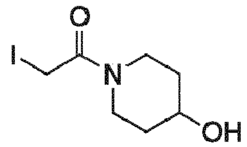

In a flame-dried flask equipped with a reflux condenser, 2-chloro-1-(4-hydroxycyclohexyl)-ethan-1-one[4] (200 mg, 1.13 mmol, 1.00 eq.) was dissolved in MeCN (dry, 3.5 mL) and NaI (762 mg, 5.08 mmol, 4.5 eq.) was added. The mixture immediately turned cloudy yellow and was stirred for 3 h at 60 °C. After completion of the reaction, water (6.0 mL) was added, and MeCN was removed under reduced pressure. EtOAc (6.0 mL) was added and the phases were separated. The aqueous phase was extracted with EtOAc (3x). The combined yellow organic layers were dried over $MgSO_4$. After filtration of the drying agent, the solvent was evaporated under reduced pressure. The crude brown oil was purified by column chromatography (95% EtOAc/pentane) to afford 2-iodo-1-(4-hydroxycyclohexyl)ethan-1-one as a dark brown oil.
HRMS ESI(+) (MeOH) calculated for $[M+H]^+$: 269.99855, found: 269.99826.

*Compounds (10a) and (10b)*

A flame-dried flask was charged with fidaxomicin (30 mg, 0.0284 mmol, 1.0 eq.) and $K_2CO_3$ (15.7 mg, 0.114 mmol, 4.0 eq.) under argon atmosphere. 2-Iodo-1-(4-hydroxycyclohexyl)-ethan-1-one (11.5 mg, 0.0426 mmol, 1.5 eq.) was added and the solids were dissolved in dry DMF (1.2 mL). The brown reaction mixture was stirred at 45 °C for 9 h. The reaction mixture was diluted with EtOAc (1.0 mL) and quenched with sat. aq. $NH_4Cl$ solution (1.0 mL). The layers were separated and the aqueous phase was extracted with EtOAc (3x). The combined organic phases were dried over $MgSO_4$ and the solvent was evaporated. The crude mixture of mono-and disubstituted products was purified by RP-HPLC [*Gemini NX* C18, 10 µ, 110 Å, 250 mm x 21.2 mm, solvent A: $H_2O$+0.1 % FA, solvent B: MeCN+0.1 % FA; 20 mL/min; LC time program (min - % B): 2.0 min - 40 %, 45.0 min - 45 %, 46.0 min - 100 %] to afford, after lyophilization, 10a ($t_R$=30.2 min, 10.3 mg, 0.009 mmol, 30%) and 10b ($t_R$=16.0 min, 8.8 mg, 0.007 mmol, 23%) as colourless solids.
10a: HRMS ESI(+) (MeOH) calculated for $[M+Na]^+$: 1220.49342, found: 1220.49393.
10b: HRMS ESI(+) (MeOH) calculated for $[M+Na]^+$: 1361.57240, found: 1361.57256.

*6-Fluoro-7-[4-(2-bromoacetyl)piperazin-1-yl}-4-oxo-1,4-dihydroquinoline-3-carboxylic acid (Bromoacetyl ciprofloxacin)*

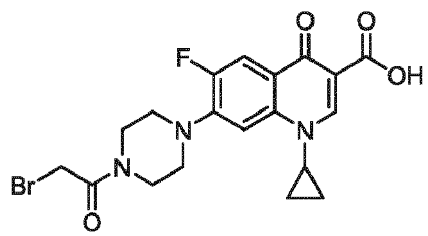

In a flame-dried flask, ciprofloxacin (250 mg, 0.754 mmol, 1.0 equiv.) was dissolved in dry THF (0.5 mL). Pyridine (61 µL, 60 mg, 0.754 mmol, 1.0 equiv.) and bromoacetylbromide (65 µL, 152 mg, 0.754 mmol, 1.0 equiv.) were added. The orange mixture was stirred at room temperature for 18 hours. Upon addition of ice to the reaction mixture, the product precipitated. The yellow solid was filtered and washed with water (3 x 5 mL). The filtrate was extracted with DCM (3 x 10 mL) and dried over $MgSO_4$. After filtration of the drying agent, the solvent was evaporated. The two portions of the crude product, *i.e.* the yellow material obtained by filtration and by extraction with DCM, were combined and purified by column chromatography (DCM/MeOH 95/5) to afford bromoacetylciprofloxacin as a yellow solid (186 mg, 0.411 mmol, 55 %).
**HRMS** ESI(+) (MeOH) calculated for $[M+H]^+$: 452.06157, found: 452.06162.

*Compound (11a) and (11b)*

An oven-dried 5 mL flask was charged with fidaxomicin (20 mg, 18.9 µmol, 1.0 eq.), $K_2CO_3$ (10.4 mg, 75.6 µmol, 4.0 eq.) and bromoacetyl ciprofloxacin (11.1 mg, 24.6 µmol, 1.3 eq.) under an atmosphere of argon. The solids were dissolved in dry DMF (0.8 mL). The milky yellow mixture was stirred at 45 °C under argon atmosphere for four

hours. The reaction mixture was filtered over kieselguhr (Celite) and washed with MeOH/MeCN (1:1). The crude product was purified by RP-HPLC *[Gemini* N*X* C18*,* 5 μ, 110 Å, 250 mm x 21.2 mm, solvent A: $H_2O$+0.1 % FA, solvent B: MeCN+0.1 % FA; 20 mL/min; LC time program (min - % B): 0.0 min - 5 %, 10.0 min - 5 %, 12 min - 60 %, 60 min - 70 %) to afford the two products as colourless solids after lyophilization (11 a: $t_R$ = 24.1 min, 10.0 mg, 0.007 mmol, 37 %; 11b: $t_R$ = 25.6 min, 6.6 mg, 0.004 mmol, 20 %).
11a: HRMS ESI+ (MeOH), calculated for $[M-H]^-$: 1426.54608, found: 1426.54661.
11b: HRMS ESI+ (MeOH), calculated for $[M+2H]^{2+}$: 900.34802, found: 900.34890.

*2,5,8,11,14,17-Hexaoxanonadecan-19-yl 4-nitrobenzenesulfonate*

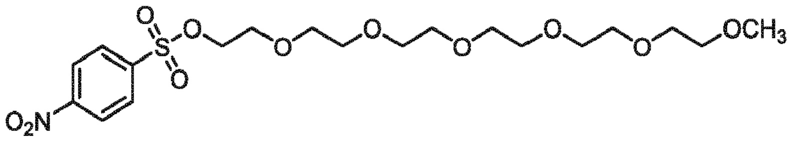

In a flame-dried 10 mL Schlenk tube, hexaethyleneglycol monomethyl ether (163 mg, 0.55 mmol, 1 eq.) was dissolved in THF (dry, 0.3 mL). Triethylamine (150 μl, 112 mg, 1.10 mmol, 2 eq.) and *N,N*-dimethylaminopyridine (16.3 mg, 0.133 mmol, 0.13 eq.) were added and the flask was cooled in an ice bath. A solution of nosyl chloride (222 mg, 1.1 mmol, 1.8 eq.) in THF (1.0 mL) was added slowly by syringe. After the addition, the reaction mixture was stirred at 0°C for five minutes and then allowed to warm to room temperature. After 18 hours of stirring at room temperature, the reaction mixture was filtered and the solid residue washed with THF (3 x 10 mL). The combined THF solutions were concentrated under reduced pressure. The oily residue was dissolved in DCM (15 mL) and washed with brine (15 mL). The aqueous phase was extracted with DCM (3 x 15 mL). The crude product was further purified by column chromatography (DCM/MeOH 96/4) to give the product as a yellow oil (239 mg, 0.50 mmol, 90 %).
**HRMS** ESI(+) (MeOH) calculated for $[M+H]^+$: 482.16906, found: 482.16923.

*Compound (12a) HEG-Fidaxomicin and (12b) Bis(HEG)-Fidaxomicin*

A flame-dried Schlenk tube was charged with fidaxomicin (58 mg, 0.055 mmol, 1.0 eq.) and the solid dissolved in DMF (dry, 0.3 mL). 2,5,8,11,14,17-Hexaoxanonadecan-19-yl 4-nitrobenzenesulfonate (186 mg, 0.386 mmol, 7.0 eq.) was dissolved in DMF (dry, 0.7 mL) and this solution was added to the reaction flask *via* microliter syringe. Solid potassium carbonate (31 mg, 0.22 mmol, 4.0 eq.) was added and washed down with another portion of DMF (dry, 0.4 mL). The reaction mixture was stirred at 45 °C. The reaction was monitored by analyzing aliquot samples by UHPLC-MS. After 2.5 hours, the reaction mixture was diluted with EtOAc (10 mL) and washed with ammonium chloride solution (15 mL). The aqueous phase was extracted with EtOAc (2x10 mL) and the combined organic phases were dried over magnesium sulfate. After filtration of the drying agent, the solvent was evaporated. The crude product was subjected to preparative RP-HPLC [Synergi Hydro-RP, 10 μ, 80 Å, 250 mm x 21.2 mm, solvent A: $H_2O$ +0.1 % FA, solvent B: MeCN+0.1 % FA; 25 mL/min; LC time program (min - % B): 4.0 min - 47 %, 52.0 min - 53 %, 58.0 min - 80 %, 58.1 min - 100 %, 63.00 min - 100 %; $\lambda$ = 270 nm] to give the monosubstitution product 12a as a colourless solid ($t_R$ = 32.54-35.33 min, 14.3 mg, 0.011 mmol, 19 %) and the disubstitution product 12b as a colourless resin ($t_R$ = 32.54-35.33 min, 41.2 mg, 0.026 mmol, 46 %).
12a: HRMS ESI+ (MeOH), calculated for $[M+Na]^+$: 1357.58738, found: 1357.58787.
12b: HRMS ESI+ (MeOH), calculated for $[M+Na]^+$: 1635.76032, found: 1635.75986.

*Compounds (15a) and (15b)*

To a sample of bis-allyl-carbonate protected fidaxomicin (94.8 mg, 0.08 mmol, 1 eq.) in a two-necked flask under argon was added Ag(OTf) (103.8 mg, 0.40 mmol, 5.0 eq.) and the flask was evaporated and flushed with argon several times. The solids were then dissolved in chloroform (dry, stabilized with amylenes, 6 ml) and the flask cooled to 0°C in an icebath. 2,6-Di-tert-butylpyridine (270 μl, 1.63 mmol, 20 eq.) and methyl iodide (25 μl, 0.4 mmol, 5 eq.) were added. The reaction mixture was allowed to warm to room temperature. After 20 hours, the reaction mixture was worked up by filtering it over a pad of Celite and washing with DCM. This solution washed with ammonium chloride solution (2x10 ml) and brine (10 ml). The crude product was subjected to column chromatography (pentane/ethyl acetate 1/1) to give the mono- and the dimethylation product (monomethylation product 15a: 20.0 mg, 0.017 mmol, 21 %; dimethylation product 15b: 45.3 mg, 0.038 mmol, 47 %; total yield: 68 %; 77 % brsm).

15a: HRMS ESI+ (MeOH), calculated for $[M+Na]^+$: 1199.4720, found: 1199.4733; Melting Point 110-111 °C.
15b: HRMS ESI+ (MeOH), calculated for $[M+Na]^+$: 1213.4876, found: 1213.4875.

*Compound (16)*

In a flame-dried Schlenk flask under argon, NaH (60 % in paraffin oil, 3.5 mg 60% NaH, 2.1 mg pure NaH, 0.0875 mmol) was dissolved in ethylene glycol (dry, 3.5 mL). Monomethylated protected fidaxomicin (40 mg, 0.034 mmol, 1 eq.) was dissolved in dry THF (1.8 mL) and the reaction mixture was cooled to 0 °C. The pre-cooled alkoxide solution was added via microliter syringe (120 μL, 0.003 mmol NaH, 0.072 mg NaH, 10 mol-%). The reaction was monitored by TLC (pentane/acetone 5/2). After 23 min, an aqueous ammonium chloride solution (10 mL) was added to the reaction mixture. After addition of ethyl acetate (10 mL), the phases were separated. The organic phase was washed with an aqueous ammonium chloride solution (3 x 10 mL) and the combined aqueous phases were extracted with ethyl acetate (2 x 30 mL). The combined organic phases were dried over magnesium sulfate. After filtration of the drying agent, the solvent was removed *in vacuo.* The crude material was subjected to column chromatography (pentane/acetone 5/2). The desired product was isolated together with a regioisomer (22.2 mg, 0.019 mmol, 57 %, mixture of regioisomers). The regioisomers were only separated after the final deprotection stage.
16: HRMS ESI+ (MeOH), calculated for $[M+Na]^+$: 1173.49269, found: 1173.49283.

*Compound (17)*

In a flame-dried Schlenk flask under argon, NaH (60 % in paraffin oil, 3.5 mg 60% NaH, 2.1 mg pure NaH, 0.0875 mmol) was dissolved in ethylene glycol (dry, 3.5 mL). Dimethylated bisallyl-protected fidaxomicin (70.7 mg, 0.06 mmol, 1 eq.) was dissolved in dry THF (2 mL) and the reaction mixture was cooled to 0 °C. The pre-cooled alkoxide solution was added via microliter syringe (0.1 mL, 0.0025 mmol, 0.06 mg, 0.04 eq.). The reaction was monitored by TLC (pentane/acetone 5/2). After conversion was completed (15 min), an aqueous ammonium chloride solution (15 mL) was added to the reaction mixture. Ethyl acetate (15 mL) was added and the phases were separated. The organic phase was washed with ammonium chloride solution (3 x 15 ml) and the combined aqueous phases were extracted with ethyl acetate (2 x 20 ml). The combined organic phases were dried over magnesium sulfate. After filtration of the drying agent, the solvent was removed in vacuo. The crude material was subjected to column chromatography (pentane/acetone 5/2) to give the desired product (15 mg, 0.013 mmol, 21 %).
17: HRMS ESI+ (MeOH), calculated for $[M+Na]^+$: 1187.50834, found: 1187.50847.

*Compound (20) Bisallyl-fidaxomicin with carbonate and TBS protecting group*

TBS(OTf) (90 μl, 104 mg, 0.39 mmol) and 2,6-lutidine (87 μl, 80 mg, 0.75 mmol) were mixed in dry DCM (0.6 ml) (c = 0.50 mmol/ml). To a stirred solution of bisallyl-fidaxomicin carbonate (236 mg, 0.20 mmol, 1 eq.) in DCM (8 mL) at 0°C, the pre-cooled solution of TBS(OTf) and lutidine in DCM (c = 0.5 mmol/ml TBS(OTf), 0.52 ml, 0.26 mmol TBS(OTf), 1.3 eq, and 0.50 mmol 2,6-lutidine, 2.5 eq.) was added slowly via syringe. The colorless solution was stirred at 0°C for 15 minutes and was then allowed to warm to room temperature. After 3.5 h, the reaction mixture was quenched with an aqueous solution of ammonium chloride (15 ml) and diluted with DCM (10 ml). The phases were separated and the aqueous phase extracted with DCM (2x10 ml). The combined organic phases were dried over magnesium sulfate. After filtration of the drying agent, the solvent was evaporated. The crude product was subjected to column chromatography (pentane/EtOAc 3/2) to give the desired product as a colourless solid (177.6 mg, 0.14 mmol, 69 %).
20: HRMS ESI(+) calculated for $[M-H]^-$: 1299.5428, found: 1299.5427.

*Compound (21a)*

An oven-dried microwave flask was charged with protected fidaxomicin (41 mg, 0.03 mmol, 1.0 eq.). The flask was evaporated and flushed with argon several times via a syringe needle. The solid was dissolved in chloroform (dry; 1.0 ml). Methyl iodide (20 μl, 45.6 mg, 0.3 mmol, 10 eq.), Ag(OTf) (40.3 mg, 0.16 mmol, 4.9 eq.) and 2,6-di-tert-butylpyridine (110 μl, 129 mg, 0.67 mmol, 21 eq.) were added. The reaction mixture was stirred for 20 min at room temperature and then heated to 35 °C. After 28 hours and after 2.5 days, another portion of MeI was added (40 μl, 91.2 mg, 20 eq., 40 μl, 91.2 mg, 20 eq.). After stirring for 3.5 days, the reaction mixture was worked up by filtering it over a pad of Celite and washing with DCM. The mixture was concentrated on the rotary evaporator, DCM was added (30 ml) and this solution washed with ammonium chloride solution (2x20 ml). The desired product was obtained as a colourless solid upon flash column (pentane/acetone 5/1) (20.7 mg, 0.02 mmol, 50 %; 75 % brsm).
21a: HRMS ESI+ (MeOH), calculated for $[M+H]^+$: 1291.57649, found: 1291.57870.

*Compound (21b) 7-Methoxybisallylfidaxomicin carbonate*

A 5 mL microwave vial under an atmosphere of argon was charged with a solution of 7-methoxy-18-TBS-bisallylfidaxomicin carbonate in dry THF (0.25 mL). Triethylamine trihydro-fluoride (75 μL, 72.9 mg of reagent, 0.167 mmol HF) was added *via* plastic syringe. The reaction mixture was heated to 50 °C for 15 hours. A diluted aqueous solution of $NaHCO_3$ (saturated solution/water 1/3, 5 mL) was added to the reaction mixture and the aqueous phase extracted with EtOAc (3 x 5 mL). The combined organic phases were washed with water (2 x 10 mL). The combined aqueous phases were extracted one more time with EtOAc (20 ml). The combined organic phases were dried over magnesium sulfate. After filtration of the drying agent, the solvent was evaporated. The crude product was subjected to preparative RP-HPLC *[Gemini NX* C18, 10 μ, 250 mm x 21.2 mm; solvents: $H_2O$+0.1 % FA; MeCN+0.1 % FA; 20 mL/min; LC time program (min - % MeCN): 0.0 - 5 %, 10.0 - 5 %, 12.0 - 65 %, 50.0 - 95 %, 51.0 - 100 %; $\lambda$ = 270 nm; $t_R$ = 45.5 min] to afford the desired product after lyophilization (4.0 mg, 0.008 mmol, 43 %).
21b: HRMS ESI+ (MeOH), calculated for [M+Na]+: 1199.47196, found: 1199.47170.

*Compound (21c) 7,18-Dimethoxybisallylfidaxomicin*

In a flame-dried Schlenk flask under argon, NaH (60 % in paraffin oil, 3.5 mg 60 % NaH, 2.1 mg pure NaH, 0.0875 mmol) was dissolved in ethylene glycol (dry, 3.5 mL). Dimethylated bisallyl-protected fidaxomicin (70.7 mg, 0.06 mmol, 1 equiv.) was dissolved in dry THF (2 mL) and the reaction mixture was cooled to 0 °C. The pre-cooled alkoxide solution was added via microliter syringe (0.1 mL, 0.0025 mmol, 0.06 mg, 0.04 equiv.). The reaction was monitored by TLC (pentane/acetone 5/2). After conversion was completed (15 min), an aqueous ammonium chloride solution (15 mL) was added to the reaction mixture. Ethyl acetate (15 mL) was added and the phases were separated. The organic phase was washed with ammonium chloride solution (3 x 15 mL) and the combined aqueous phases were extracted with ethyl acetate (2 x 20 mL). The combined organic phases were dried over magnesium sulfate. After filtration of the drying agent, the solvent was removed in vacuo. The crude material was subjected to column chromatography (pentane/acetone 5/2) to give the desired product (15 mg, 0.013 mmol, 21 %).
21c: HRMS ESI+ (MeOH), calculated for $[M+Na]^+$: 1187.50834, found: 1187.50847.

*Compound (21d) 7-Methoxybisallylfidaxomicin*

Preparation of the alkoxide solution: In a flame-dried Schlenk flask under argon, NaH (60 % in paraffin oil, 8.6 mg 60% NaH, 5.16 mg pure NaH, 0.215 mmol) was dissolved in ethylene glycol (dry, 4.3 mL; c(NaH) = 0.05 mmol/mL).

Carbonate deprotection: 7-Methoxybisallylfidaxomicin (5.7 mg, 4.84 umol, 1.0 equiv.) was dissolved in THF (dry, 0.16 mL) and the reaction mixture was cooled to 0 °C. The NaH-ethylene glycol solution (pre-cooled) was added *via* syringe (193 μL, 9.68 umol, 2 equiv. NaH). After 30 min, a saturated aqueous solution of ammonium chloride (2 mL) was added to the reaction mixture. After addition of ethyl acetate (2 mL), the phases were separated. The organic phase was washed with ammonium chloride solution (3 x 2 mL) and the combined aqueous phases were extracted with ethyl acetate (2 x 8 mL). The combined organic layers were dried over magnesium sulfate. After filtration of the drying agent, the solvent was removed *in vacuo.* The crude product was purified by column chromatograpy (EtOAc/pentane 1:1) to give the desired product as a colourless solid (1.8 mg, 0.005 mmol, 32 %).
21d: HRMS ESI+ (MeOH), calculated for [M+Na]+: 1173.49269, found: 1173.49264.

*3-Azidopropan-1-ol*

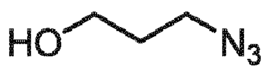

3-Azidopropan-1-ol was synthesized following a literature procedure: A. W. Gann, J. W. Amoroso, V. J. Einck, W. P. Rice, J. J. Chambers, N. A. Schnarr, Org. Lett. 2014, 16, 2003-2005. In a 500 mL round-bottom flask, 3-bromopropan-1-ol (12.4 g, 89.2 mmol, 1 eq.) was dissolved in acetone (200 mL). A solution of sodium azide (29.0 g, 466 mmol, 5.2 e.) in deionized water (120 mL) was added while stirring. A catalytic amount of KI (approximately 2 mg) was added. This solution was stirred at room temperature for 48 hours. After extraction with diethyl ether (3 x 80 mL), the combined organic layers were dryed over $K_2CO_3$. The drying agent was filtered off and the solvent evaporated *in vacuo* to give 3-azidopropan-1-ol as a highly viscous colourless oil (8.09 g, 80.0 mmol, 90 %).
HRMS ESI(+) (MeOH) calculated for $[M+Na]^+$: 124.04813, found: 124.04790.

*3-Azidopropyl 4-nitrobenzenesulfonate*

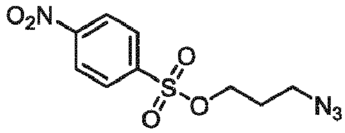

3-Azidopropyl 4-nitrobenzenesulfonate was synthesized in analogy to a nosylation procedure reported in literature: J. Bucher, T. Wurm, K. S. Nalivela, M. Rudolph, F. Rominger, A. S. K. Hashmi, Angew. Chem. Int. Ed. 2014, 53, 3854-3858. In a flame-dried 250 mL three-necked round-bottom flask, 3-azidopropan-1-ol (2.50 g, 24.7 mmol, 1 eq.) was dissolved in THF (dry, 30 mL). DMAP (453 mg, 3.70 mmol, 0.15 eq.) and $NEt_3$ (6.95 mL, 45.5 mmol, 1.8 eq.) were added. The reaction mixture was cooled to 0 °C and a solution of nosyl chloride (11.0 g, 49.5 mmol, 2 eq.) in THF (100 mL, dry) was added slowly via a dropping funnel. As large amounts of precipitate had formed, another portion of THF (dry, 50 mL) was added. The reaction mixture was stirred at room temperature for 48 hours. The mixture was diluted with DCM (700 mL), washed with brine (3 x 200 mL), dried over anhydrous $MgSO_4$, filtered and concentrated *in vacuo.* After purification by flash column chromatography ($SiO_2$, *n*-pentane/DCM 1/3) the product (4.7 g, 16.5 mmol, 67 %) was obtained as a slightly yellow amorphous solid.
HRMS ESI(+) (MeOH) calculated for $[M+Na]^+$: 286.03739, found: 286.03774.

*Compounds (22a) and (22b)*

Fidaxomicin (101.4 mg, 95.8 μmol, 1 eq.) was weighed into a flame-dried 5 mL Schlenk tube under nitrogen atmosphere and dissolved in DMF (dry, 1 mL). 3-Azidopropyl 4-nitrobenzenesulfonate (40.9 mg, 143 μmol, 1.5 eq.) and $K_2CO_3$ (60.8 mg, 0.44 mmol, 4 eq.) were added and washed down with more DMF (dry, 1 mL). The reaction mixture was stirred at 45 °C for 3 hours. The reaction mixture was diluted with EtOAc (10 mL), washed with a saturated aqueous solution of ammonium chloride (5 x 10 mL) and dried over $MgSO_4$. After filtration of the drying agent, the solvent was evaporated to give a yellow oil. The crude product was purified by preparative RP-HPLC [*Gemini NX* C18, 10 μ, 110 Å, 250 mm x 21.2 mm; solvent A: $H_2O$+0.1 % FA, solvent B: MeCN+0.1 % FA; 20 mL/min; LC time program (min - % MeCN): 0.0 - 60 %, 3.0 - 60 %, 30.0 - 85 %; $\lambda$ = 270 nm) to afford the desired products after lyophilization (monosubstituted product 22a: $t_R$ = 19.3 min, 20.4 mg, 0.018 mmol, 19 %; disubstituted product 22b: $t_R$ = 26.9 min, 33.7 mg, 0.028 mmol, 29 %).
<u>22a:</u> HRMS ESI+ (MeOH), calculated for $[M+Na]^+$: 1162.46279, found: 1162.46327.
<u>22b:</u> HRMS ESI+ (MeOH), calculated for $[M+Na]^+$: 1245.51114, found: 1245.50954.

*Compounds* (23a) *and (23b)*

A flame-dried Schlenk tube under an atmosphere of argon was charged with fidaxomicin (70 mg, 0.066 mmol, 1.0 equiv.). 3-Azidopropyl 4-nitrobenzenesulfonate (23 mg, 0.081 mmol, 1.2 equiv.) was added. The flask was evacuated and flushed with argon several times. The two solids were dissolved in DMF (dry, 1.0 mL). Solid potassium carbonate (37 mg, 0.27 mmol, 4.0 equiv.) was added and washed down with another portion of DMF (1.5 mL). The reaction mixture was stirred at 40 °C; after two hours, the reaction temperature was raised to 45 °C. After 4.5 hours, the reaction mixture was diluted with ethyl acetate (15 mL) and an aqueous solution of ammonium chloride (20 mL) was added. The aqueous phase was extracted with ethyl acetate (15 mL) and the combined organic phases were washed with ammonium chloride solution (3x15 mL) and dried over magnesium sulfate. After filtration of the drying agent, the solvent was evaporated.

The dinuclear CuAAC catalyst (9.6 mg, 0.014 mmol) was weighed into an oven-dried Schlenk flask under an atmosphere of argon. The crude mixture from the preceding reaction containing fidaxomicin as well as mono(azidopropyl)- and bis(azidopropyl)fidaxomicin was dissolved in DCM (2.0 mL) and added to the catalyst. Glacial acetic acid (100 %, 11 μl, 12 mg, 0.19 mmol) and 3-ethynylaniline (23 mg, 0.19 mmol; distilled prior to use at 110 °C oil bath temperature and 3 mbar) were dissolved in DCM (0.8 mL) and added to the reaction mixture, which turned bright yellow upon addition of the alkyne solution. After 4 hours, the reaction mixture was diluted with DCM (dry, 10 mL), washed with brine (3 x 10 mL) and dried over $MgSO_4$. The solvent was removed *in vacuo.* The product mixture was separated by preparative RP-HPLC [*Gemini NX* C18, 5 μ, 110 Å, 250 mm x 21.2 mm; solvent A: $H_2O$ +0.1 % FA, solvent B: MeCN+0.1 % FA; 20 mL/min; LC time program (min - % B): 5.0 min - 40 %, 36.0 min - 65 %, 39.0 min - 80 %, 39.1 min - 100 %; $\lambda$ = 270 nm] to give the monotriazole product 23a ($t_R$ = 22.6-25.8 min, 30.2 min, 18 mg, 0.014 mmol, 22 % over two steps) and the bistriazole 23b ($t_R$ = 26.5-28.8 min, 19 mg, 0.013 mmol, 19 % over two steps) as well as recovered fidaxomicin starting

material ($t_R$ = 31.2 min, 17.2 mg, 0.011 mmol, 17 %) as colourless solids.
23a: HRMS ESI- (MeOH), calculated for $[M-H]^-$: 1255.52414, found: 1255.52560.
23b: HRMS ESI+ (MeOH), calculated for $[M+H]^+$: 1457.64489, found: 1457.64631.

*Compounds (24a) and (24b) (SulfonylF-Fidaxomicin)*

An oven-dried microwave tube under an atmosphere of argon was charged with fidaxomicin (52 mg, 0.049 mmol, 1.0 equiv.) and 4-(bromomethyl)benzene sulfonyl fluoride (22 mg, 0.088 mmol, 1.8 equiv.). The solids were dissolved in DMF (dry, 1.5 mL) and potassium carbonate (27 mg, 0.20 mmol, 4.0 equiv.) was added. The reaction mixture was stirred at 45 °C. After two hours, the reaction mixture was diluted with diethyl ether (15 mL) and quenched with saturated ammonium chloride solution (15 mL). The layers were separated and the aqueous phase extracted with diethyl ether (4 x 15 mL). The organic phase was washed with ammonium chloride solution (2 x 50 mL). The combined organic phases were dried over magnesium sulfate. After filtration of the drying agent, the solvent was evaporated. The crude product was subjected to preparative RP-HPLC *[Gemini NX* C18*,* 5 μ, 110 Å, 250 mm x 21.2 mm; solvent A: $H_2O$ +0.1 % FA, solvent B: MeCN+0.1 % FA; 20 mL/min; LC time program (min - % B): 4.0 min - 60 %, 35.0 min - 85 %, 45.1 min - 100 %; $\lambda$ = 270 nm] to give the monosubstitution product **A (24a)** ($t_R$ = 22.53-23.56 min, 11 mg, 0.0088 mmol, 16 %) and the disubstitution product **B (24b)** ($t_R$ = 32.39-34.17 min, 32 mg, 0.023 mmol, 47 %) as colourless solids.
24a: HRMS ESI+ (MeOH), calculated for $[M+Na]^+$: 1251.41497, found: 1251.41380.
24b: HRMS ESI+ (MeOH), calculated for $[M+NH_4]^+$: 1418.45790, found: 1418.45885.

*5-Propynylacetamide fluorescein*

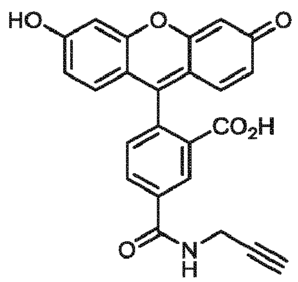

Chemical Formula: $C_{24}H_{15}NO_6$; Exact Mass: 413.0899; Molecular Weight: 413.3850

5-Propynylacetamide fluorescein was synthesized following a literature procedure.[2] In a flame-dried 5 mL Schlenk tube, 5-carboxyfluorescein (65.1 mg, 173.1 μmol, 1.0 equiv.) was dissolved in anhydrous DMF (2 mL, dry). HOBt (46.2 mg, 341.3 μmol, 2.0 equiv.) and triethylamine (34.2 mg, 337.1 μmol, 1.9 equiv.) were added while stirring at room temperature. *N,N'*-Diisopropylcarbodiimide (42.2 mg, 335.0 μmol, 1.9 equiv.) was added and the mixture was stirred for 20 minutes at room temperature. Propargylamine (14.5 mg, 263.3 μmol, 1.5 equiv.) was added and the reaction mixture was stirred for 26 hours. The mixture was diluted with 1 M HCl (5 mL) and extracted with DCM (5 x 10 mL). The combined organic phases were washed with a saturated aqueous solution of sodium chloride (2 x 10 mL), dried over anhydrous $MgSO_4$, filtered and concentrated *in vacuo.* After purification by flash column chromatography ($SiO_2$, DCM/MeOH 98/2 → 96/4 → 10/1), the product (54.8 mg, 133.2 μmol, 77 %) was obtained as a bright orange solid.
**HRMS** ESI+ (MeOH), calculated for $[M+H]^+$: 414.0973, found: 414.0972.

*Compound (25) (Monotriazole with Fluorescein)*

A flame-dried Schlenk-tube under nitrogen was charged with a solution of 5-propynylacetamide-fluoresceine (6.7 mg, 16.2 μmol, 1.3 equiv.) in *t*-BuOH/$H_2O$ (1:1, 1.4 mL, degassed) and (3-azidopropyl)fidaxomicin (14.0 mg, 12.3 μmol, 1.0 equiv.) was added. Upon addition of Cu(I)OAc (3.4 mg, 27.7 μmol, 2.3 equiv.), the reaction mixture turned yellow and was stirred at room temperature under nitrogen atmosphere for four hours. The reaction mixture was then diluted with MeOH, filtered through kieselguhr (Celite), and the solvent was evaporated. The crude product was purified by silica gel column chromatography (DCM/MeOH 9/1 to 4/1) to afford the desired compound as an orange solid (12.2 mg, 0.008 mmol, 64 %).
25: HRMS ESI+ (MeOH), calculated for $[M+H]^+$: 1553.57078, found: 1553.57163.

*Compound of general formula (26a) (Monotriazole with Piperidin-4-amine Substituent)*

In an oven-dried Schlenk-tube, to a solution of fidaxomicin-monoazide (22.0 mg, 19.3 μmol, 1.0 equiv.) and 1-(but-3-yn-1-yl)piperidine-4-amine dihydrochloride (8.7 mg, 38.6 μmol, 1.3 equiv.) in *t*-BuOH/$H_2O$ (1:1, degassed, 2.0 mL) was added Cu(I)OAc (1.20 mg, 9.65 μmol, 0.5 equiv.) was added. This mixture was stirred at room temperature for 30 h under argon atmosphere. The solvent was evaporated and the obtained solid was dissolved in MeOH/$H_2O$ (70/30) and filtered over an SPE-C18 cartridge. The product was further purified by RP-HPLC *[Gemini NX* C18*,* 5μm, 110 Å, 250 mm x 21.2 mm; solvent A: $H_2O$+0.1% FA, solvent B: MeCN+0.1% FA; 20 mL/min; LC time program (min - % B): 0.0 - 5 %, 30.0 - 95 %, 31.0 - 100%] to afford, after lyophilization, the product ($t_R$=17.2 min, 14.2 mg, 0.011 mmol, 57 %) as a colourless solid.
26a: HRMS ESI+ (MeOH), ), calculated for $[M+H]^+$: 1292.61219, found: 1292.61307.

*Compound (26b) (Bistriazole with Piperidine-4-amine Substituent)*

An oven-dried Schlenk tube was charged with a solution of bis(3-azidopropyl)fidaxomicin (15.2 mg, 12.4 μmol, 1.0 equiv.) and 1-(but-3-yn-1-yl)piperidine-4-amine dihydrochloride (7.0 mg, 31.1 μmol, 2.5 equiv.) in *t*-BuOH/$H_2O$ (1:1, degassed, 1.4 mL). Cu(I)OAc (0.45 mg, 3.67 μmol, 0.3 equiv.) was added and the reaction mixture stirred at room temperature for 24 hours under an atmosphere of argon. The solvent was evaporated and the obtained solid was dissolved in MeOH/$H_2O$ (3/2) and filtered over an SPE-C18 cartridge. The product was purified by preparative RP-HPLC *[Gemini NX* C18*,* 5 μ, 110 Å, 250 mm x 21.2 mm; solvent A: $H_2O$+0.1 % FA, solvent B: MeCN+0.1 % FA; 20 mL/min; LC time program (min - % B): 0.0 - 5 %, 5.0 - 5 %, 30.0 - 95 %, 31.0 - 100 %] to afford, after lyophilization, the product ($t_R$=15.0 min, 8.2 mg, 5.0 μmol, 43 %) as a colourless solid.
26b: HRMS ESI+ (MeOH), calculated for [M+2H]2+: 764.39959, found: 764.39950.

*Compound (27) (Monotriazole with PEG5-acid)*

A flame-dried 5 mL flask was charged with a solution of alkyne-PEG5-acid (5.98 mg, 19.7 μmol, 1.5 equiv.) in a solvent mixture of tBuOH/$H_2O$ (1:1, degassed by freeze-pump-thaw cycling, 1.5 mL). 3-Azidopropylfidaxomicin (15.0 mg, 13.1 μmol, 1.0 equiv.) as well as solid Cu(OAc) (1.0 mg, 8.16 μmol, 0.6 equiv.) were added. The mixture was stirred at room temperature under an atmosphere of argon for 48 hours. It was diluted with DCM (5 mL) and washed with brine (3 x 5 mL). The combined organic phases were dried over anhydrous $MgSO_4$. After filtration of the drying agent, the solvent was evaporated under reduced pressure. The product was purified by preparative RP-HPLC [*Gemini NX* C18, 5 μ, 110 A, 250 mm x 21.2 mm; solvents: $H_2O$+0.1 % FA; MeCN+0.1 % FA; 20 mL/min; LC time program (min - % B): 0.0 - 5 %, 10.0 - 5 %, 12.0 - 55 %, 50.0 - 65 %, 51.0 - 100 %; $\lambda$ = 270 nm; $t_R$ = 20.8 min] to afford the desired product after lyophilization (4.1 mg, 0.013 mmol, 22 %).
27: HRMS ESI+ (MeOH), calculated for [M-H]-: 1442.61849, found: 1442.61987.

*1-Nitrosopiperazine*

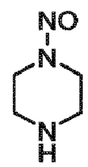

1-Nitrosopiperazine was synthesized following a literature procedure.[3] In a flame-dried 500 ml round-bottom flask piperazine (10.32 g, 119.81 mmol) was dissolved in 6 M HCl (72 mL) and cooled to -10 °C. A solution of $NaNO_2$ (8.26 g, 119.81 mmol) in deionized water (144 mL) was added dropwise by dropping funnel over 1 hour. After completion of the addition, the reaction mixture was adjusted to pH = 10 with 3 M NaOH (80 mL) and extracted with $CHCl_3$ (5 x 75 mL). The combined organic phases were dried over anhydrous $MgSO_4$, filtered and concentrated *in vacuo.* After purification by flash column chromatography ($SiO_2$, DCM/MeOH 92/8), the product (4.05 g, 35.61 mmol, 29 %) was obtained as a yellowish oil.
**HRMS** ESI(+) (MeOH) calculated for $[M+H]^+$: 116.0818, found: 116.0819.

*1-Nitroso-4-propargylpiperazine[3]*

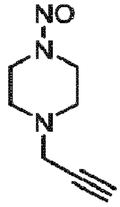

1-Nitroso-4-propargylpiperazine was synthesized following a literature procedure.[3] In a flame-dried 50 ml round-bottom flask, 1-nitrosopiperazine (1.53 g, 13.31 mmol) was dissolved in anhydrous MeCN (36 ml). Propargyl bromide (1.45 ml, 2.01 g, 13.52 mmol) and $NEt_3$ (3.70 ml, 26.62 mmol) were added and the reaction mixture was heated under reflux conditions for 5 hours. Reaction control after 5 h by TLC indicated only sluggish conversion. Therefore, another portion of propargyl bromide (400 mg, 2.68 mmol, 0.2 eq.) was added and the reaction mixture was stirred for 16 hours under reflux conditions. The reaction mixture was concentrated *in vacuo,* dissolved in 10 % NaOH (100 ml) and extracted with DCM (3 x 40 ml). The combined organic phases were dried over anhydrous $MgSO_4$, filtered and concentrated *in vacuo.* After purification by flash column chromatography ($SiO_2$, DCM/MeOH 96/4) the product (1.34 g, 8.74 mmol, 66 %) was obtained as a beige solid.
**HRMS** ESI(+) (MeOH) calculated for $[M+H]^+$: 154.0974, found: 154.0976.

*1-Amino-4-propargylpiperazine*

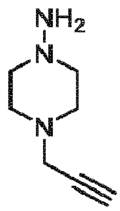

1-Amino-4-propargylpiperazine was synthesized following a literature procedure with a slightly different workup.[3] In a flame-dried 250 ml round-bottom flask, $LiAlH_4$ (228 mg, 6.0 mmol, 2.0 equiv.) was suspended in anhydrous $Et_2O$ (80 ml) and was stirred vigorously. A solution of 1-nitroso-4-propargylpiperazine (460 mg, 3.0 mmol, 1.0 equiv.) in anhydrous $Et_2O$ (20 ml) was added and the reaction mixture was stirred under reflux conditions for three hours. The reaction mixture was cooled to 0 °C and a saturated solution of sodium potassium tartrate (100 ml, aq.) was added slowly. After completion of the addition, the mixture was vigorously stirred for three hours, then filtered and extracted with DCM (3 x 100 ml). The combined organic phases were dried over anhydrous $MgSO_4$, filtered and concentrated in vacuo. After purification by flash column chromatography ($SiO_2$, DCM/MeOH 10/1) the product (104 mg, 740 μmol, 25 %) was obtained as an off-white solid.
HRMS ESI(+) (MeOH) calculated for $[M+H]^+$: 140.1182, found: 140.1182.

*Alkinylated Rifampicin*

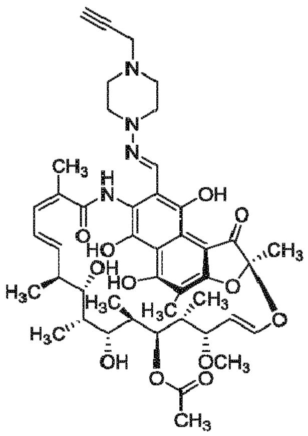

Alkinylated rifampicin was synthesized following a literature procedure.[3] In a flame-dried 5 ml Schlenk tube, 3-formylrifamycin SV (100 mg, 138 μmol) was dissolved in anhydrous THF (2 mL, dry) and 1-amino-4-propargylpiperazine (21.1 mg, 151.8 μmol, ) was added. Additional anhydrous THF (2 mL, dry) was added to the reaction mixture and it was stirred at room temperature for 15 minutes. It was diluted with DCM (10 mL), washed with a saturated aqueous solution of sodium chloride (2 x 10 mL) and the combined organic phases were dried over anhydrous $MgSO_4$. After filtration of the drying agent and evaporation of the solvent, the product **6** was obtained as a bright red solid (106 mg, 90 μmol, 91 %, containing slight impurities).
**HRMS** ESI(+) (MeOH) calculated for $[M+H]^+$: 847.41240, found: 847.41231.

*23-Azido-3,6,9,12,15,18,21-heptaoxatricosyl 4-nitrobenzenesulfonate*

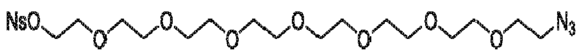

In a flame-dried 10 ml Schlenk tube, O-(2-azidoethyl)heptaethylene glycol (215 mg, 0.545 mmol, 1.0 equiv.) was dissolved in DCM (dry, 2.0 mL). Triethylamine (150 μL, 109 mg, 1.08 mmol, 2.0 equiv.) and DMAP (15 mg, 0.13 mmol, 0.23 equiv.) were added and the flask was cooled in an ice bath. Nosyl chloride (241 mg, 1.08 mmol, 2.0 equiv.) in DCM (1.0 mL) was added by syringe and washed down with more DCM (1.0 mL). The reaction mixture was stirred at 0 °C for five minutes and then allowed to warm to room temperature. After stirring at room temperature for 22 hours, the reaction mixture was poured on an aqueous solution of ammonium chloride (20 mL) and the phases were separated. The aqueous phase was extracted with DCM (3 x 15 mL). The combined organic phases were dried over magnesium sulfate. After filtration of the drying agent and evaporation of the solvent, the crude product was purified by preparative RP-HPLC *(Gemini NX* C18, 10 μ, 110 Å, 250 mm x 21.2 mm; solvent A: $H_2O$+0.1 % FA, solvent B: MeCN+0.1 % FA; 20 mL/min; LC time program (min - % B): 4.0 min - 45 %, 36.0 min - 80 %, 46.0 min - 100 %; $\lambda$ = 270 nm] to afford the desired product ($t_R$ = 12.2 min, 168 mg, 0.289 mmol, 53 %) after lyophilization.
**HRMS** ESI(+) (MeOH) calculated for $[M+NH_4]^+$: 598.23887, found: 598.23907.

*Compound (28) (Fiadxomicin-OEG-Azide)*

A flame-dried Schlenk tube under an atmosphere of argon was charged with fidaxomicin (84.0 mg, 0.079 mmol, 1.0 equiv.) and a solution of 23-azido-3,6,9,12,15,18,21-heptaoxa-tricosyl 4-nitrobenzenesulfonate (68.6 mg, 0.12 mmol, 1.5 equiv.) in DMF (dry, 1 mL) was added by microliter syringe. Potassium carbonate (44.5 mg, 0.32 mmol, 4.1 equiv.) was added and washed down with DMF (dry, 1.0 mL). The reaction mixture was stirred for 21.75 hours at 45 °C under an atmosphere of argon. The reaction mixture was diluted with ethyl acetate (15 mL) and quenched with saturated ammonium chloride solution (15 mL). The layers were separated and the aqueous phase extracted with ethyl acetate (2 x 15 mL). The combined organic phases were dried over magnesium sulfate. After filtration of the drying agent, the solvent was evaporated. The crude product was subjected to preparative HPLC [*Gemini NX* C18, 5 μ, 110 Å, 250 mm x 21.2 mm; solvent A: $H_2O$+0.1 % FA, solvent B: MeCN+0.1 % FA; 20 mL/min; LC time program (min - % B): 4.0 min - 60 %, 35.0 min - 85 %, 45.1 min - 100 %; $\lambda$ = 270 nm] to afford the desired product ($t_R$ = 17.6 min, 39.9 mg, 0.289 mmol, 35 %) after lyophilization.
28: HRMS ESI(+) (MeOH) calculated for [M+2NH4]2+: 734.85454, found: 734.85350.

*Compound (29) (Fidaxomicin-Rifampicin Hybrid)*

A 10 mL one-necked flask containing fidaxomicin-OEG-azide (21 mg, 0.015 mmol, 1.0 equiv.), was charged with rifampicin alkyne (15 mg, 0.017 mmol, 1.2 equiv.) and the flask was evacuated and flushed with argon several times. The solids were dissolved in DCM (dry, 0.7 mL). The CuAAC dicopper catalyst was added as solid (2.4 mg, 0.003 mmol, 0.23 equiv.) and washed down with another portion of DCM (dry, 0.1 mL). After 2.5 hours, another portion of dicopper catalyst (3.4 mg, 0.005 mmol, 0.33 equiv.) was added. After three hours, the reaction mixture was diluted with DCM (5 mL) and poured on a saturated aqueous solution of ammonium chloride (5 mL). The phases were separated and the aqueous phase was extracted with DCM (3 x 5 mL). The combined organic phases were dried over magnesium sulfate. After filtration of the drying agent and evaporation of the solvent, the crude product was purified by preparative RP-HPLC [*Phenomenex Synergi Hydro-RP,* 10 μ, 80 Å, 250 mm x 21.2 mm, solvent A: $H_2O$+0.1 % FA, solvent B: MeCN+0.1 % FA; 20 mL/min; LC time program (min - % B): 0.0 min - 55 %, 4.00 - 55 %, 44.0 min - 70 %, 50.0 min - 85 %, 50.1 min - 100 %] to yield the bright red solid product after lyophilisation (8.6 mg, 0.0038 mmol, 26 %).
29: HRMS ESI(+) (MeOH) calculated for [M+NH4+H]2+: 1149.54382, found: 1149.54424.

***Antibacterial testing of fidaxomicin derivatives***

Bacterial strains

| Organism | Strain |
|---|---|
| *Bacillus subtilis* | DSM3256 |
| *Staphylococcus aureus* | ATCC29213 |
| *Mycobacterium tuberculosis* H37Ra | ATCC25177 |

The strains of *Bacillus subtilis* and *Staphylococcus aureus* were grown overnight at 37 °C on MH II agar plates. (BD™ BBL™ Mueller Hinton II Agar, BD Diagnostics). MIC values were determined by broth dilution method according to the recommendations of the Clinical and Laboratory Standards Institute (CLSI; U.S.A.). The inoculum size was about $7.5 \times 10^5$ colony forming units/well. The compounds were diluted in $H_2O$ from 1.0 mg/mL stock solutions in methanol/$H_2O$ 1/1 in a 2-fold dilution series. The microtiter plates were incubated at 37 °C overnight. Afterwards, the MIC (lowest concentration of the compounds with no bacterial growth observed) was determined by visual inspection.

An *M. tuberculosis* H37Ra (ATCC25177) liquid culture (5 mL, Middlebrook 7H9 broth base + 10 % ADC supplement) was inoculated from original stock (-80°C). After two to five days of shaking at 37°C, an $OD_{600}$ (optical density at $\lambda$ = 600 nm) of approximately 1 was observed. The MIC was determined directly from the adjusted liquid culture ($OD_{600}$ = 0.53, $3.3 \times 10^6$ colony forming units/well, 0.6 McFarland units)[4] by applying two serial dilution series of the test compounds in $H_2O$ from stock solutions (1mg/mL in methanol/$H_2O$ 1/1; rifampicin from 0.32 mg/mL stock in $H_2O$). Plates were covered with plastic foil and a lid. MIC values were determined by visual inspection or by plate reader at 600 nm after 3-14 days (pre-tempered at 37°C with plastic foil, lid removed).

Table 1: The MIC values in Table 1 are given in μg/mL and in nmol/mL.

| Compound | *Staphylococcus aureus* | *Bacillus subtilis* | *Mycobacterium tuberculosis* H37Ra |
|---|---|---|---|
| Fidaxomicin (positive control) | 8-16 μg/mL 7.6-15.1 nmol/mL | 8-16 μg/mL 7.6-15.1 nmol/mL | 4 μg/mL 3.8 nmol/mL |
| Compound 5a (Fidaxomicin Ethylacetate) | 16 μg/mL 14.0 nmol/mL | 32 μg/mL 28.0 nmol/mL | 2-8 μg/mL 1.8-7.0 nmol/mL |
| Compound 5b (Fidaxomicin Bis (ethylacetate)) | 32-64 μg/mL 26.0-52.0 nmol/mL | 8-16 μg/mL 6.5-13.0 nmol/mL | 1-4 μg/mL 0.8-3.3 nmol/mL |
| Compound 7a (Fidaxomicin Piperazine with alloc Protecting Group) | 16-32 μg/mL 12.6-25.2 nmol/mL | >64 μg/mL >50.5 nmol/mL | |
| Compound 26b (Ditriazole with Piperidin-4-amine Substituent) | >64 μg/mL >41.9 nmol/mL | 8-16 μg/mL 5.2-10.5 nmol/mL | 4-16 μg/mL 2.6-10.5 nmol/mL |

(continued)

| Compound | *Staphylococcus aureus* | *Bacillus subtilis* | *Mycobacterium tuberculosis* H37Ra |
|---|---|---|---|
| Compound 11a (Fidaxomicin-Ciprofloxacin Hybrid) | >64 µg/mL >44.8 nmol/mL | 32->64 µg/mL 22.4->44.8 nmol/mL | 16-64 µg/mL 11.2-44.8 nmol/mL |
| Compound 29 (Fidaxomicin-Rifampicin Hybrid) | 4 µg/mL 1.8 nmol/mL | 2-32 µg/mL 0.9-14.0 nmol/mL | 0.5-2 µg/mL 0.2-0.9 nmol/mL |

***Determination of Aqueous Solubility***

*Calibration with Methanol Solutions*

A standard stock solution of each analyte in MeOH at a concentration of 1.0 mg/mL was prepared. All analytes were completely soluble in MeOH. A standard stock solution of caffeine for use as internal standard (IS) in MeOH was prepared at a concentration of 1 mg/mL. These stock solutions were further diluted with MeOH to calibration standard samples at concentrations of 50, 100, 200, 300, 400, 500 µg/mL of analyte with each sample containing 50 µg/mL of caffeine as IS. These samples were analyzed by UHPLC [*Kinetex*® EVO C18; 1.7 µm; 100 Å, 50 mm x 2.1 mm; *Phenomenex;* solvent A: $H_2O$ + 0.1 % HCOOH, solvent B: MeCN + 0.1 % HCOOH; 0.4 mL/min; 1.0 µL injection volume, LC time program (min - % B): 0.50 min - 5 %, 3.50 min - 95 %, 3.55 min - 100 %; UV detection at $\lambda$ = 270 nm]. The peak area of the analyte signal was divided by the peak area of the IS. This peak ratio was plotted against the analyte's concentration to obtain a linear equation.

*Determination ofAqueous Solubility*

For reproducibility reasons, water solubility of the fidaxomicin derivatives was quantified in a pH = 7 phosphate buffered aqueous solution (Acros, CAS 7558-79-4). A stock solution of caffeine in phosphate buffer at a concentration of 1.0 mg/mL was prepared and diluted to a concentration of 50 µg/mL. To 1.0 mL of this caffeine solution, the analyte was added as a solid until a saturated solution was obtained. This solution was stirred for five hours at room temperature. The mixture was filtered over a 0.22 µm syringe filter and the obtained solution was analyzed by UHPLC. The concentration was then calculated from the linear equation obtained from the calibration.

With most samples of fidaxomicin derivatives, two peaks for the analyte were detected, *viz*. one for the original substance and one for its regioisomer formed by transacylation of the isobutyric acid ester. In the following table, the total solubility of each analyte is given with the percentage of isomerization added in parentheses (weight-% of transacylated isomer), assuming that both regioisomers feature approximately the same absorption characteristics.

Table 2: Aqueous solubility of fidaxomicin derivatives

| Compound | Conc. [µg/mL] (weight-% of isomer) |
|---|---|
| Fidaxomicin | 84 (9 %) |
| Compound 10b (AMM-212 F5-7) | 213(10%) |
| Compound 10a (AMM-212 F14-15) | 180 (7 %) |
| Compound 26b (AMM-308 F5) | 451 (12%) |
| Compound 12b (rb273 f17-20) | 504 (49 %) |
| Compound 27 (AMM-382 F6) | >2148* |
| * No isomer peak detected: Saturation not reached with the material available. | |

Literature References

[1] G. R. Fulmer, A. J. M. Miller, N. H. Sherden, H. E. Gottlieb, A. Nudelman, B. M. Stoltz, J. E. Bercaw, K. I. Goldberg,

Organometallics 2010, 29, 2176-2179.
[2] M. A. Brun, K.-T. Tan, R. Griss, A. Kielkowska, L. Reymond, K. Johnsson, J. Am. Chem. Soc. 2012, 134, 7676-7678.
[3] S. A. Cochrane, X. Li, S. He, M. Yu, M. Wu, J. C. Vederas, J. Med. Chem. 2015, 58, 9779-9785.
[4] K. Peñuelas-Urquides, L. Villarreal-Treviño, B. Silva-Ramírez, L. Rivadeneyra-Espinoza, S. Said-Fernández, M. B. de León, Braz. J. Microbiol. 2013, 44, 287-290.

## Claims

**1.** A compound **characterized by** a general formula (1):

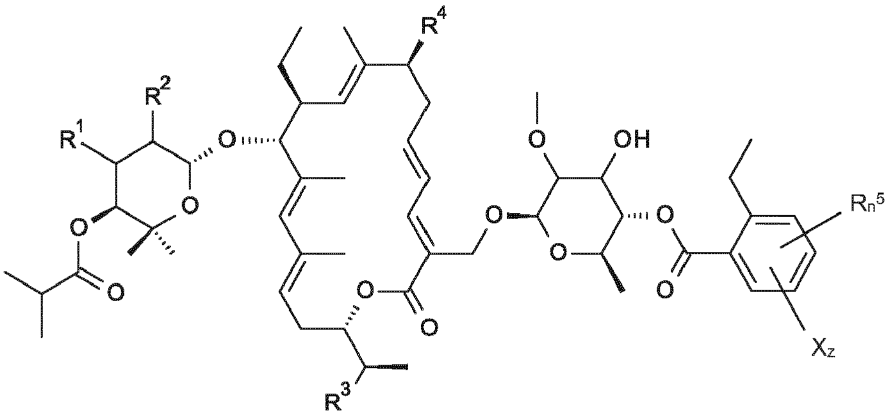

(1),

wherein

- $R^1$ and $R^2$ are independently from each other $C_{1-4}$-alcohol, -OH, -O-$R^k$ or -O-C(=O)-$R^k$, or $R^1$ and $R^2$ together are -O-C(=O)-O-, with $R^k$ being a $C_{1-4}$-alkyl or $C_2$-$_4$-alkenyl,
- $R^3$ and $R^4$ are independently from each other $C_{1-6}$-alcohol, -OH, $C_{1-6}$-alkoxy, silyl ether, in particular tert-butyldimethylsilyl ether (TBS), -$NR^iH$, -$NR^iR^j$, $P_{1-6}$-phosphane, phenol, -O-C(=O)-$R^i$, -OCN, with $R^i$ and $R^j$ being independently from each other a $C_{1-4}$-alkyl or $C_2$-$_4$-alkenyl, wherein $R^3$ and $R^4$ are unsubstituted or substituted by $C_1$-$_4$-alkyl or a $C_1$-$_4$-alkyl substituted with at least one -F, -Cl, -Br or -I,
- n of $R_n^5$ is 1 or 2, and each $R^5$ is independently from each other $R^5$
-OH, or
-O-$L_n$-$R^a_p$-$R^b_q$-$L_t$-$R^a_r$- $R^b_s$-$R^e$, in particular -O-$L_n$-$R^a_p$-$R^b_q$-$L_tR^a_r$-$R^e$ or -O-$L_n$-$R^a_p$-$R^b_q$-$L_t$-$R^b_s$-$R^e$, or
-O-$L_n$-$R^b_q$-$R^d$-$L_tR^b_s$-$R^e$
with

  - n, p, q, r, s and t being independently from each other 0 or 1,
  - each L being independently from each other L selected from $C_1$-$_{12}$-alkyl, in particular $C_1$-$_6$-alkyl, more particularly $C_1$-$_3$-alkyl,
  - $R^a$ being independently from each other $R^a$ -(C=O)-, -(C=O)-NH-, -(C=O)-$NR^e$-, -NH-(C=O)-, -(C=O)-O- or -O-(C=O)-,
  - $R^b$ being independently from each other $R^b$ a three to six membered heterocycle or heteroaryl, a $C_{3-6}$-cycloalkyl or $C_{6-14}$-aryl,
  - $R^d$ is a polyether linker comprising m elements -$C_u$-alkyl-O-, wherein u is independently selected for each element from an integer between 1 and 4 and m is an integer between 1 and 12, in particular 1 and 8,
  - $R^e$ being -H, -$C_{1-4}$-alkyl, -$C_{2-4}$-alkenyl, -$C_{2-4}$-alkynyl, -OH, -COOH, -$NH_2$, -$N_3$, -$SO_2F$, -F, -Cl, -Br, -I, a dye, in particular a fluorescent dye, more particularly fluorescein, or an antibiotic, in particular ciprofloxacin or rifampicin,

- z of $X_z$ is 1 or 2, and each $X_z$ is independently from each other $X_z$ -CN, -$CF_3$, - $CH_2F$, -$CHF_2$, -$CH_2$-$CF_3$, -$CH_2$-$CH_2F$, -$CH_2$-$CHF_2$, -$OR^h$, -$SR^h$, -$SOR^h$, -$SO_2R^h$, $SO_2$-NH-$R^h$, -F, -Cl, -Br or -I, wherein $R^h$ is a $C_1$-$_4$-alkyl or a $C_1$-$_4$-alkyl substituted with at least one -F, -Cl, -Br or -I,

with the proviso that the compound is not fidaxomicin.

**2.** The compound according to claim 1, wherein the compound is **characterized by** general formula (2),

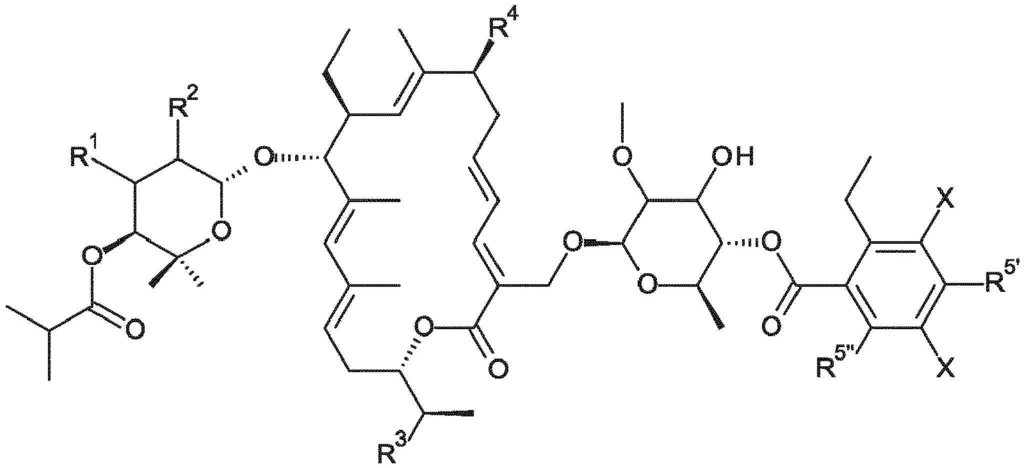

(2),

with $R^{5'}$ and $R^{5''}$ having independently from each other the same meaning as defined for $R^5$ and $R^1$,$R^2$, $R^3$, $R^4$, $R^5$ and X having the same meaning as defined above.

**3.** The compound according to any one of the preceding claims, wherein $R^{5'}$ and $R^{5''}$ are independently from each other -OH, $-O-L-R^a-R^e$, $-O-R^e$, $-O-L-R^a-R^b-R^e$, $-O-L-R^a-R^b-R^a-R^e$, $-O-L-R^e$, $-O-L-R^b-R^b-R^e$, - $O-L-R^b-R^e$, $-O-L-R^b-L-R^b-R^e$, $-O-L-R^b-L-R^b-R^e$, $-O-R^d-R^e$, $-O-L-R^b-R^d-L-R^e$, $-O-R^d-L-R^e$, or $-O-R^d-L-R^b-R^e$, in particular -OH, $-O-L-R^a-R^e$, $-O-L-R^a-R^b-R^e$, $-O-L-R^a-R^b-R^a-R^e$, $-O-L-R^e$, $-O-L-R^b-R^b-R^e$, $-O-L-R^b-R^e$, $-O-L-R^b-L-R^b-R^e$, $-O-L-R^b-L-R^b-R^e$, $-O-R^d-R^e$, $-O-L-R^b-R^d-L-R^e$, $-O-R^d-L-R^e$, or $-OR^d-L-R^b-R^e$, more particularly -OH, $-O-L-R^a-R^b,R^e$, $-O-L-R^b-L-R^b-R^e$, $-O-(R^d)_m-R^e$, or $-O-L-R^b-R^d-L-R^e$, $-O-L-R^a-R^b-R^a-R^e$, $-O-L-R^b-R^b-R^e$, $-O-(R^d)_m-L-{}_R{}^e$.

**4.** The compound according to any one of the preceding claims, wherein

- one of $R^{5'}$ and $R^{5''}$, in particular $R^{5'}$, is selected from $-O-L-R^a-R^e$, $-O-R^e$, $-O-L-R^a-R^b-R^e$, $-O-L-R^a-R^b-R^a-R^e$, $-O-L-R^e$, $-O-L-R^b-R^b-R^e$, $-O-L-R^b-R^e$, $-O-L-R^b-L-R^a-R^e$, $-O-L-R^b-L-R^b-R^e$, $-O-R^d-R^e$, $-O-L-R^b-R^d-L-R^e$, $-O-R^d-L-R^e$ or $-O-R^d-L-R^b-R^e$, in particular from $-O-L-R^a-R^e$, $-O-L-R^a-R^b-R^e$, $-O-L-R^a-R^b-R^a-R^e$, $-O-L-R^e$, $-O-L-R^b-R^b-R^e$, $-O-L-R^b-R^e$, $-O-L-R^b-L-R^a-R^e$, $-O-L-R^b-L-R^b-R^e$, $-O-R^d-R^e$,$-O-L-R^b-R^d-L-R^e$, $-O-R^d-L-R^e$ or $-O-R^d-L-R^b-R^e$, more particularly $-O-L-R^a-R^b-R^e$, $-O-L-R^b-L-R^b-R^e$, $-O-R^d-R^e$, $-O-L-R^b-R^d-L-R^e$, $-O-L-R^a-R^b-R^a-R^e$, $-O-L-R^b-R^b-R^e$, or $-O-R^d-L-R^e$, and
- the other one of $R^{5'}$ and $R^{5''}$, in particular $R^{5''}$, is -OH, or
- both $R^{5'}$ and $R^{5''}$ are independently from each other selected from $-O-L-R^a-R^e$, $-O-R^e$,$-O-L-R^a-R^b-R^e$, $-O-L-R^a-R^b-R^a-R^e$, $-O-L-R^e$, $-O-L-R^b-R^b-R^e$, $-O-L-R^b-R^e$, $-O-L-R^b-L-R^b-R^e$, $-O-L-R^b-L-R^b-R^e$, $-O-R^d-R^e$, $-O-L-R^b-R^d-L-R^e$ $-O-R^d-L-R^e$ or $-O-R^d-L-R^b-R^e$, in particular from $-O-L-R^a-R^e$, $-O-L-R^a-R^b-R^e$, $-O-L-R^a-R^b-R^a-R^e$, $-O-L-R^e$, $-O-L-R^b-R^e$, $-O-L-R^b-R^e$, $-O-L-R^b-L-R^b-R^e$, $-O-L-R^b-L-R^b-R^e$, $-O-R^d-R^e$,$-O-L-R^b-R^d-L-R^e$, $-O-R^d-L-R^e$ or $-O-R^d-L-R^b-R^e$, more particularly $-O-L-R^a-R^b-R^e$, $-O-L-R^b-L-R^b-R^e$, $-O-R^d-R^e$, $-O-L-R^b-R^d-L-R^e-O-L-R^a-R^b-R^a-R^e$, $-O-L-R^b-R^b-R^e$, or $-O-R^d-L-R^e$.

**5.** The compound according to any one of the preceding claims, wherein $R^1$ and $R^2$ are independently from each other -OH, or $-O-C(=O)-R^k$, or $R^1$ and $R^2$ together are -O-C(=O)-O-, in particular $R^1$ and $R^2$ are independently from each other -OH or $-O-C(=O)-R^k$, with $R^k$ being a $C_{1-4}$-alkyl, in particular isopropyl, more particularly $R^1$ and $R^2$ are -OH.

**6.** The compound according to any one of the preceding claims, wherein $R^3$ and $R^4$ are independently from each other -OH, $-O-(CH_2)_{1-4}-CH_3$, in particular -OH, $-O-CH_2-CH_3$ or $-OCH_3$, more particularly-OH.

**7.** The compound according to any one of the preceding claims, wherein $R^a$ of $R^5$ is - (C=O)-, -(C=O)-NH-, -NH-(C=O)-, -(C=O)-O-, in particular-(C=O)-.

**8.** The compound according to any one of the preceding claims, wherein L is $C_1$-$_6$-alkyl, in particular $C_{1-3}$-alkyl.

**9.** The compound according to any one of the preceding claims, wherein $R^b$ of $R^5$ is a five to six membered heterocycle, a $C_{5-6}$-cycloalkyl or $C_6$-aryl, in particular a five to six membered heterocycle, more particularly piperazine, piperidine, tetrazole, triazole, imidiazole or $C_6$-aryl even more particularly piperazine, piperidine or triazole.

**10.** The compound according to any one of the preceding claims, wherein $R^e$ of $R^5$ is -H, - $C_{1-2}$-alkyl, -$C_{2-4}$-alkenyl, allyl, -OH, -COOH, -$NH_2$, -$N_3$, -$SO_2F$, ciprofloxacin, fluorescein or rifampicin, in particular -H, $C_{1-2}$-alkyl, -OH, -COOH, -$NH_2$,-$N_3$ or -$SO_2F$, more particularly -H, $C_{1-2}$-alkyl, -OH, -COOH or -$NH_2$.

**11.** The compound according to any one of the preceding claims, wherein m is 4 to 8, in particular 5 to 7, and/or the elements of $R^d$ are selected from -$(CH_2)_{1-4}$-O-, in particular -$(CH_2)_{1-2}$-O-, more particularly each element $R^d$ is -$(CH_2)_2$-O- or the first element of $R^d$ is -$CH_2$-O- and the other elements of $R^d$ are -$(CH_2)_2$-O-.

**12.** The compound according to any one of the preceding claims, wherein z of $X_z$ is 1 or 2, in particular 2, and each $X_z$ is independently from each other $X_z$ -$CF_3$, -O-$CF_3$, -S-$CF_3$, -SO-$CF_3$, -$SO_2CF_3$, $SO_2$-NH-$CF_3$, -F, -Cl, -Br or -I, in particular -F, - Cl, -Br or -I, more particularly -Cl.

**13.** The compound according to any one of the preceding claims, wherein the solubility in phosphate-buffered water (pH = 7) is at least 40 μg/ml, in particular >90 μg/ml, more particularly > 150 μg/ml, even more particularly >1500 μg/mL.

**14.** A compound according to any one of the preceding claims for use in a method of treatment of disease.

**15.** A compound according to any one of claims 1 to 12 for use in a method of treatment of infections, in particular bacterial infections, more particularly bacterial infections caused by

- *Clostridioides,* in particular *Clostridium difficile* or *Clostridium perfringens,*
- *Mycobacteria,* in particular *Mycobacterium tuberculosis,*
- *Staphylococci,* in particular *Staphylococcus aureus,*
- *Enterococci,* in particular *Enterococcus faecium,*
- *Escherichia,* in particular *Escherichia coli,* and
- *Bacilli,* and/or

for use in a method of treatment of bacterial infections caused by drug resistant bacteria, in particular

• resistant *Mycobacterium tuberculosis,*
• resistant *Staphylococcus* species, in particular methicillin-resistant *Staphylococcus aureus,*
• resistant *Enterococci,* in particular vancomycin-resistant *Enterococci.*

Europäisches Patentamt
European Patent Office
Office européen des brevets

# EUROPEAN SEARCH REPORT

Application Number
EP 18 15 0671

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br>Y | WO 96/35702 A1 (ABBOTT LAB [US]) 14 November 1996 (1996-11-14)<br>* abstract; claims *<br>* page 6, line 18 - page 7, line 23 *<br>----- | 1-6,8, 10,12-15<br>1-15 | INV.<br>C07H17/08<br>A61K31/7048<br>A61P31/04 |
| X | ELIAS KAUFMANN ET AL: "Total Synthesis of the Glycosylated Macrolide Antibiotic Fidaxomicin", ORGANIC LETTERS, vol. 17, no. 14, 17 July 2015 (2015-07-17), pages 3514-3517, XP055485827, DOI: 10.1021/acs.orglett.5b01602<br>* page 3516; compound 25 *<br>----- | 1-6,8,10 | |
| Y | WO 2016/004166 A1 (XAVIER UNIVERSITY OF LOUISIANA [US]) 7 January 2016 (2016-01-07)<br>* abstract *<br>* claims 1, 45; compound 26 *<br>----- | 1-15 | |
| A | WO 2009/070779 A1 (OPTIMER PHARMACEUTICALS INC [US]; ICHIKAWA YOSHI [US]; CHIU YU-HUNG [U) 4 June 2009 (2009-06-04)<br>* abstract; claim 1 *<br>* page 5; compounds OP-1438 *<br>----- | 1-15 | TECHNICAL FIELDS SEARCHED (IPC)<br>C07H<br>A61K<br>A61P |
| | -/-- | | |

The present search report has been drawn up for all claims

1

| Place of search | Date of completion of the search | Examiner |
|---|---|---|
| Munich | 19 June 2018 | Gohlke, Pascale |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 15 0671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | REVILL P ET AL: "Tiacumicin B", DRUGS OF THE FUTURE , vol. 31, no. 6 31 December 2006 (2006-12-31), pages 494-497, XP002696172, ISSN: 0377-8282, DOI: 10.1358/DOF.2006.031.06.1000709 Retrieved from the Internet: URL:http://journals.prous.com/journals/servlet/xmlxsl/dof/20063106/pdf/df310494.pdf?p_JournalId=2&p_refId=1000709&p_IsPs=N [retrieved on 2013-04-25] * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 19 June 2018 | Gohlke, Pascale |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 18 15 0671

This annex lists the patent family members relating to the patent documents cited in the above-mentioned European search report. The members are as contained in the European Patent Office EDP file on
The European Patent Office is in no way liable for these particulars which are merely given for the purpose of information.

19-06-2018

| Patent document cited in search report | Publication date | Patent family member(s) | Publication date |
|---|---|---|---|
| WO 9635702 A1 | 14-11-1996 | AT 203246 T | 15-08-2001 |
| | | CA 2218916 A1 | 14-11-1996 |
| | | DE 69613975 D1 | 23-08-2001 |
| | | DE 69613975 T2 | 04-04-2002 |
| | | DK 0824539 T3 | 24-09-2001 |
| | | EP 0824539 A1 | 25-02-1998 |
| | | ES 2159733 T3 | 16-10-2001 |
| | | GR 3036873 T3 | 31-01-2002 |
| | | JP H11504937 A | 11-05-1999 |
| | | PT 824539 E | 30-01-2002 |
| | | US 5583115 A | 10-12-1996 |
| | | WO 9635702 A1 | 14-11-1996 |
| WO 2016004166 A1 | 07-01-2016 | CN 106715446 A | 24-05-2017 |
| | | EP 3164405 A1 | 10-05-2017 |
| | | JP 2017522375 A | 10-08-2017 |
| | | US 2017137443 A1 | 18-05-2017 |
| | | WO 2016004166 A1 | 07-01-2016 |
| WO 2009070779 A1 | 04-06-2009 | EP 2222309 A1 | 01-09-2010 |
| | | ES 2554364 T3 | 18-12-2015 |
| | | JP 5843344 B2 | 13-01-2016 |
| | | JP 2011504938 A | 17-02-2011 |
| | | TW 200932250 A | 01-08-2009 |
| | | US 2010035833 A1 | 11-02-2010 |
| | | US 2012040922 A1 | 16-02-2012 |
| | | US 2013252912 A1 | 26-09-2013 |
| | | WO 2009070779 A1 | 04-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS,* 85721-33-1
- *CHEMICAL ABSTRACTS,* 13292-46-1
- *CHEMICAL ABSTRACTS,* 2321-07-5
- *CHEMICAL ABSTRACTS,* 873857-62-6
- **A. W. GANN ; J. W. AMOROSO ; V. J. EINCK ; W. P. RICE ; J. J. CHAMBERS ; N. A. SCHNARR.** *Org. Lett.,* 2014, vol. 16, 2003-2005
- **J. BUCHER ; T. WURM ; K. S. NALIVELA ; M. RUDOLPH ; F. ROMINGER ; A. S. K. HASHMI.** *Angew. Chem. Int. Ed.,* 2014, vol. 53, 3854-3858
- *CHEMICAL ABSTRACTS,* 7558-79-4
- **G. R. FULMER ; A. J. M. MILLER ; N. H. SHERDEN ; H. E. GOTTLIEB ; A. NUDELMAN ; B. M. STOLTZ ; J. E. BERCAW ; K. I. GOLDBERG.** *Organometallics,* 2010, vol. 29, 2176-2179
- **M. A. BRUN ; K.-T. TAN ; R. GRISS ; A. KIELKOWSKA ; L. REYMOND ; K. JOHNSSON.** *J. Am. Chem. Soc.,* 2012, vol. 134, 7676-7678
- **S. A. COCHRANE ; X. LI ; S. HE ; M. YU ; M. WU ; J. C. VEDERAS.** *J. Med. Chem.,* 2015, vol. 58, 9779-9785
- **K. PEÑUELAS-URQUIDES ; L. VILLARREAL-TREVIÑO ; B. SILVA-RAMÍREZ ; L. RIVADENEYRA-ESPINOZA ; S. SAID-FERNÁNDEZ ; M. B. DE LEÓN ; BRAZ. J.** *Microbiol.,* 2013, vol. 44, 287-290